# EUROPÄISCHE PATENTANMELDUNG

(11) **EP 2 151 253 A1**
(43) Veröffentlichungstag der Anmeldung: **10.02.2010**
(21) Anmeldenummer: 08161598.1
(22) Anmeldetag: 31.07.2008
(51) Int. Cl.: A61L 27/34, A61L 29/08, A61L 31/10, C23C 16/30, C23C 16/42

(54) **Biokompatibilitätsschicht und beschichtete Gegenstände**

(71) Anmelder: Fraunhofer-Gesellschaft zur Förderung der angewandten Forschung e.V., 80686 München (DE); Bio-Gate AG, 28359 Bremen (DE)
(72) Erfinder: Ott, Matthias, 21255, Dohren (DE); Grunwald, Ingo, 28359, Bremen (DE); Salz, Dirk, 28195, Bremen (DE); Wagener, Michael, 28355, Bremen (DE); Vissing, Klaus-Dieter, 27321, Morsum (DE); Hielscher, Wolfgang, 28755, Bremen (DE); Dölle, Christopher. Dr, 27749 Delmenhorst (DE)
(74) Vertreter: Eisenführ, Speiser & Partner

(57) **Zusammenfassung**

Die vorliegende Erfindung betrifft die Verwendung einer vernetzten siliziumhaltigen Schicht enthaltend, bestehend im wesentlichen aus oder bestehend aus Silizium, O, C, H, optional N, die durch Plasmapolymerisation und/oder Vernetzung von siliziumorganischen Flüssigkeiten mit Hilfe eines Plasmaprozesses und/oder UV-Strahlung einer Wellenlänge unter 250 nm ohne die Verwendung von Metallen einer Ordnungszahl über 14 herstellbar ist, als biokompatible Oberfläche, zum Vermitteln oder Versehen einer Oberfläche mit einer biokompatiblen Wirkung, oder zum Modifizieren einer biokompatiblen Wirkung einer Oberfläche. Ferner betrifft die Erfindung entsprechend beschichtete Gegenstände und Verfahren zu ihrer Herstellung.

## Beschreibung

Die vorliegende Erfindung betrifft die Verwendung einer vernetzten siliziumhaltigen Schicht enthaltend, bestehend im wesentlichen aus oder bestehend aus Silizium, O, C, H, optional N, die durch Plasmapolymerisation und/oder Vernetzung von siliziumorganischen Flüssigkeiten mit Hilfe eines Plasmaprozesses und/oder UV-Strahlung einer Wellenlänge unter 250 nm ohne die Verwendung von Metallen einer Ordnungszahl über 14 herstellbar ist als biokompatible Oberfläche, zum Vermitteln oder Versehen einer Oberfläche mit einer biokompatiblen Wirkung, oder zum Modifizieren einer biokompatiblen Wirkung einer Oberfläche. Ferner betrifft die Erfindung entsprechend_beschichtete Gegenstände und Verfahren zu ihrer Herstellung.

Biokompatibilität im Sinne der vorliegenden Erfindung bezeichnet allgemein die Eigenschaft eines Materials, gegenüber einem vorgewählten biologischen Zielorganismus (insbesondere einem Menschen) über einen vorgewählten Zeitraum keine den Fortbestand des Organismus oder seiner Organe gefährdenden oder seine Reproduktionsfähigkeit beeinflussenden Wirkungen zu haben. Nachfolgend bezeichnet "Biokompatibilität" insbesondere die Eigenschaft eines erfindungsgemäß beschichteten Materials, gegenüber einem Menschen und seinen Organen
- keine oder lediglich akzeptable Zytotoxizität, gemessen gemäß ISO 10993-5:2003,
- keine oder lediglich akzeptable Reizungen oder intrakutane Reaktivität, gemessen gemäß ISO 10993-10:2003,
- keine oder lediglich akzeptable systemische Toxizität (akute Toxizität, subakute Toxizität und subchronische Toxizität), gemessen gemäß ISO 10993-11:2003,
- keine oder lediglich akzeptable Genotoxizität, gemessen gemäß ISO 10993-3:2003,
- ausreichende oder hohe Implantationsverträglichkeit, gemessen gemäß ISO 10993-6:2003,
- ausreichende oder hohe Hämokompatibilität, gemessen gemäß ISO 10993-4:2003,

je nach Einsatzgebiet des erfindungsgemäß beschichteten Materials. Eine Biokompatibilitätsschicht im Sinne der vorliegenden Erfindung ist dementsprechend eine Schicht, die, wenn sie in unmittelbaren Kontakt mit lebenden Zellen, Geweben, Organen oder Lebewesen, insbesondere einem Menschen oder einer menschlichen Zelle, Gewebe oder Organ steht, Biokompatibilität gemäß einer oder mehreren der oben beschriebenen ISO-Definitionen aufweist. Erfindungsgemäß bilden Biokompatibilitätsschichten vorzugsweise Oberflächen auf Gegenständen, die bei bestimmungsgemäßer Anwendung in Berührung mit menschlichen Zellen, Gewebe oder Organen gebracht werden, wobei die in Berührung mit den Zellen, Gewebe oder Organen stehende Oberfläche des Gegenstandes zumindest teilweise mit einer Biokompatibilitätsschicht bedeckt ist. Gegenstände, die bei bestimmungsgemäßer Anwendung in Berührung mit menschlichen Zellen, Gewebe oder Organen gebracht werden, werden im Rahmen dieser Erfindung auch vereinfacht als Medizinprodukt bezeichnet.

Verschiedene Gegenstände mit biokompatibler Oberfläche sind als solche bereits bekannt. Wie nachfolgend dargelegt war es bisher jedoch aufwändig, Biokompatibilität vermittelnde Oberflächen auf diesen Gegenständen herzustellen, oder Verfahren, die bei einem bestimmten Gegenstand zur Ausbildung einer biokompatiblen Oberfläche führen, auf andere Gegenstände zu übertragen:

In verschiedenen technischen Bereichen wie der Sensortechnologie, insbesondere aber auch im Bereich von Medizinprodukten, beispielsweise bei Implantaten, Stents, Schrittmachern, Injektionssystemen oder Kathetern, spielt die Biokompatibilität der verwendeten Materialien eine wichtige Rolle. Neben der generellen guten Verträglichkeit solcher Gegenstände werden fast immer auch noch zusätzliche Eigenschaften in den jeweiligen Einsatzgebieten gefordert. Um diese Eigenschaften zu erzielen, werden herkömmlicherweise deren Oberflächen durch (nass)chemische und/oder physikalische Verfahren bearbeitet. Hierdurch soll erreicht werden, dass sich zum Beispiel Proteine an den behandelten Oberflächen besser anlagern. Ein Beispiel einer solchen Oberflächenbehandlung ist das Aufbringen geeigneter Oberflächengruppen wie z.B. Amino-, Hydroxyl- oder Carboxylgruppen. Das hierbei verfolgte Ziel ist, dass die jeweiligen behandelten Gegenstände (beispielsweise Implantate) sich leichter in vorhandene Gewebestrukturen einpassen können, indem sie beispielsweise im Falle von Implantaten fester mit Knochengewebe verbunden werden und diese Verbindung verträglicher ausfällt, insbesondere indem lokale pathologische Effekte in lokalem Gewebe auf makroskopischer und mikroskopischer Ebene begrenzt oder verringert werden. Durch eine Oberflächenbearbeitung soll in anderen Fällen auch erreicht werden, dass (Körper-) Zellen, insbesondere Knochenzellen, Gewebezellen, Endothelzellen, sich an diese Oberflächen anlagern und diese bewachsen, so dass es zu einer schnelleren Integration des Gegenstandes, z.B. des Implantats, in das umliegende Gewebe kommt. Dies ist zum einem für eine verbesserte Verankerung und Verträglichkeit wünschenswert. Zum anderen wird hierdurch die Heilung beschleunigt und das Risiko einer entzündlichen Abstoßung reduziert.

Auf der anderen Seite versucht man, bei manchen Gegenständen genau den gegenteiligen Effekt zu erreichen, also die Anlagerung von Biomolekülen und Zellen auf bestimmten Oberflächen zu vermeiden. Durch eine Anlagerung kann es zu einer unerwünschten Funktionseinschränkung z.B. im Bereich von Sensoren, aber auch zum Verstopfen von Kanülen oder Schläuchen kommen. Diese Effekte werden auch als Fouling bezeichnet. Implantate haben häufig Zonen, in denen ein Zellbewuchs unerwünscht ist. Bei Kurzzeit-Kathetern kann durch eine zu gute Gewebeintegration die einfache Entfernung erschwert werden.

Ein weiterer nachteiliger Effekt von gut mit Biomolekülen und Zellen verbindbaren Oberflächen besteht darin, dass durch eine unerwünschte Interaktion von Zellen mit den eingebrachten Materialien zellbiologische Reaktionen wie beispielsweise Entzündungsreaktionen und Zellagglomerationen induziert werden können, welche zu schwersten Nebenwirkungen führen können.

Die Besiedlung von Implantatoberflächen oder Prothesen mit Mikroorganismen, z.B. durch Antibiotika-resistente Staphylokokken, stellt ein schwerwiegendes Problem in der Medizin dar. Durch die Infektion der Patienten, respektive die Besiedlung der Implantate, kann es zu lebensbedrohenden Nebenwirkungen kommen. Diese sind oftmals nur durch einen sehr kostspieligen Ersatz des Implantates und einen langen Krankenhausaufenthalt zu begegnen. Der Bakterienadhäsion geht immer eine Adhäsion von Biomolekülen wie z.B. Proteinen aus dem Serum voraus. Geeignete anti-adhäsive Beschichtungen können hierbei die Besiedlung mit Bakterien stark reduzieren.

Zur Behandlung von Krankheiten, wie z.B. Infektionen, werden dem Patienten häufig Infusionen verabreicht oder Zugänge gelegt. Hierbei wird dem Patienten meist ein Katheter, ein Port oder eine Kanüle gesetzt. Im Bereich des Durchtritts bzw. im Körper kommt es häufig zu entzündlichen Reaktionen, die u.a. durch eine mangelnde Biokompatibilität des Materials hervorgerufen werden. Eine die Zelladhäsion fördernde Beschichtung vermag hier diese unerwünschten Reaktionen zu reduzieren.

Für die Behandlung schwerster Krankheiten, beispielsweise aggressive Tumore, Autoimmunerkrankungen oder bei Infektionen, stehen seit einigen Jahren neue Protein- und DNA-basierte Medikamente zur Verfügung. Hierbei handelt es sich z.B. um Antiköper gegen Tumorzellen, Peptidantibiotika gegen Infektionen oder siRNA gegen Autoimmunerkrankungen. Der Vorteil dieser neuen Wirkstoffe liegt u.a. darin, dass diese in relativ geringen Mengen wirksam sind. Hierbei tritt jedoch das Problem auf, dass bei der Verabreichung (z.B. intravenös) diese Substanzen an die Oberflächen der Spritzen, Schläuche oder Vorratsflaschen adsorbieren, sich also unspezifisch und ungewollt anlagern. Um den Wirkstoffverlust zu kompensieren, werden diese meist 15- 25 % höher dosiert als dies eigentlich notwendig wäre.

Eine weitere Aufgabe der Beschichtung ist es, eine nicht schädigende Interaktion einer Hohlfaser von Membran-Oxygenatoren mit Blut zu ermöglichen. Ein Oxygenator ist ein Teil einer Herz-Lungen-Maschine, die dem Blut Sauerstoff zuführt. Der Oxygenator umfasst Hohlfasern, in denen Sauerstoff hindurch geführt wird. An der äußeren Oberfläche der Fasern läuft der Blutstrom entlang. Dieser sogenannte Membran-Oxygenator tauscht Kohlendioxid gegen Sauerstoff aus.

Herkömmlicherweise sind zahlreiche Versuche unternommen worden, Oberflächen mit Fouling-verhindernden oder -verzögernden Eigenschaften (sogenannte "Anti-Fouling-Oberflächen") und/oder mit anti-adhäsiven Eigenschaften zu versehen. Einige dieser Versuche werden nachfolgend kurz beschrieben. Dabei werden auch solche Dokumente gewürdigt, deren Inhalt erst rückschauend, also in Kenntnis der fertigen vorliegenden Erfindung, als strukturell zumindest entfernt ähnlich erkannt wurde, auch wenn sie selbst keinen Hinweis zur Lösung der Aufgabe der Erfindung enthalten.

Dem Stand der Technik entsprechend können z.B. Implantatoberflächen durch nasschemische Verfahren mit Polyethylenglykol (PEG), Poly(ethylenoxid) (PEO) oder ähnlichen Polymeren beschichtet werden, um anti-fouling oder anti-adhäsive Oberflächen herzustellen.

Es ist bekannt, dass mit CHF₃-Pulsplasmen Oberflächen derartig beschichtet werden können, dass das Anhaften (Adhäsion) und das Wachstum von Zellen zu beeinflussen ist (M. Haupt, J. Barz, U. Vohrer, H. Hilgers, C. Oehr in Vakuum in Forschung und Praxis, 2005, Band 17, Ausgabe 6, Seiten 329 - 335). Ein derartiges Vorgehen ist aber mit den bekannten Problemen bei der Verwendung von fluorhaltigen Plasmen verbunden: Die Precursoren sind vergleichsweise teuer und können zu cancerogenen Zwischenprodukten führen.

Zudem ist die Abscheidung von speziellen Siliziumcarbid-Plasmabeschichtungen zur Verbesserung der Bio- und Hämokompatibilität von Stentgrundkörpern bekannt (Vortrag von Dr. Carsten Momma zum Themenkomplex" Plasmatechnik in der Gefäßmedizin" auf dem Innovationsforum "PlasmaPlusBio" am 02. Februar 2006 in Greifswald). Hierbei handelt es sich um ein sehr teures Beschichtungsverfahren, da die Beschichtungsanlagen für Hochvakuum ausgestattet sein müssen.

Die US5182317 offenbart ein Beschichtungsverfahren, bei dem eine Oberflächenbeschichtung mit Aminen funktionalisiert wird. Das Verfahren ist verhältnismäßig aufwändig und entsprechend teuer durchzuführen. Ähnlich aufwändige Verfahren sind beispielsweise aus der US5455040 bekannt.

Bei Membran-Oxygenatoren werden gegenwärtig häufig Heparinmoleküle beispielsweise über einen chemischen Prozess an die äußere Oberfläche der Faser angebunden. Jedoch hat diese Methode den Nachteil, dass sie sehr aufwändig ist und der Sauerstoff/Kohlendioxid-Austausch im Blut mit derart behandelten Fasern nur schlecht funktioniert.

Aus DE69932273 T2 ist ferner bekannt, dass Oxygenatorfasern mit Aminfunktionalitäten versehen werden können. Dieses geschieht durch Auftragung einer aminfunktionellen Polysiloxanlösung. Anschließend bringt man die so funktionalisierte Oberfläche mit einem Biomolekül in Kontakt, beispielsweise mit Heparin. Heparin soll die Bildung von Thromben verhindern, so dass die Thrombosegefahr für den Patienten sinkt. Kommerziell werden Oxygenatoren mit dieser Beschichtung unter dem Markennamen "QUADROX Bioline coated" vertrieben.

Alternativ wird mit Hilfe von Polypeptiden die Oberfläche der Oxygenatorfasern biokompatibel ausgestattet. Diese werden unter dem Handelsnamen "QUADROX Safeline treated" vertrieben.

Die US6549811 offenbart die Verwendung plasmapolymerer Beschichtungen zur Strukturierung der Oberflächen von Elastomeren wie Kathetern aus Silikon.

Die EP1060031 B1 beschreibt die Vernetzung von Silikonöl mit Plasma als Beschichtung für einen Behälter für ein pharmazeutisches Proteinpräparat. Die Beschichtung soll die Adhäsion von Proteinen an der beschichteten Oberfläche verringern.

Die WO 2005/014075 A1 offenbart allgemein eine Plasmapolymerisation mit Siloxanen zur Beschichtung von "Ausdehnungballons" zum Verbessern der Abrasionsbeständigkeit und zum Vorbeugen gegen kleinste Löcher.

Aus der EP1701676B1 ist eine Wundauflage bekannt, die eine antimikrobiell wirksame Substanz (Silber) in einem durch Plasmapolymerisation aus Hexamethyldisiloxan gebildeten Polymer besitzt. Über einen Anti-Fouling-Effekt oder anti-adhäsive Eigenschaften einer silberfreien Oberfläche ist nichts angegeben.

Die DE 103 537 56 offenbart ein antimikrobielles, nicht zytotoxisches Schichtmaterial. Das Schichtmaterial enthält Silber und eine plasmapolymere Schicht als Transportkontrollschicht. Die DE 103 537 56 offenbart jedoch nicht, dass auch ohne Anwesenheit von Silber Zytotoxizität vermieden oder gar Biokompatibilität im erfindungsgemäßen Sinne herbeigeführt werden kann. Ferner erlaubt das beschriebene Verfahren nicht bzw. nur sehr aufwändig die Beschichtung komplexer Bauteilgeometrien.

Gleichfalls zeigt die US6589546 allgemein ein Wirkstoffabgabesystem mit plasmapolymerer Deckschicht, jedoch ohne nähere Erläuterungen zu Wechselwirkungen mit Körperzellen.

Die WO 02100928 A1 offenbart verschiedene plasmapolymere Schichten, ohne jedoch einen Hinweis auf eventuelle Wechselwirkungen mit Körperzellen oder den Einsatz im direkten Kontakt mit Körpergewebe zu geben.

Aus der EP 982041 A1 sind spezielle Plasma-Beschichtungen mit funktionalisierten Siloxanen bekannt. Sie sollen zur Bildung thromboresistenter Beschichtungen medizinischer Geräte dienen. Die vorgestellten Beschichtungen sind aufwändig herzustellen.

Die DE 10031198 A1 zeigt sehr allgemein Plasma-Beschichtungen von medizintechnischen Oberflächen. Die Beschichtungen sollen als Korrosionsschutz wirken. Über eine verbesserte Biokompatibilität ist nichts Konkretes gesagt.

Ferner zeigt die EP 745220 B1 "bio-inerte" Festphasensensoren mit einer aus Silikoncarbinol gebildeten Beschichtung.

Aufgabe der vorliegenden Erfindung war es daher, den Nachteilen des Standes der Technik abzuhelfen oder diese Nachteile zu mindern und verbesserte biokompatible Beschichtungen sowie entsprechend beschichtete Gegenstände und Verwendungen der Beschichtungen anzugeben.

Die Aufgabe wird gelöst durch Angabe einer Verwendung einer vernetzten siliziumhaltigen Schicht enthaltend, bestehend im wesentlichen aus oder bestehend aus Silizium, O, C, H, optional N, die durch Plasmapolymerisation und/oder Vernetzung von siliziumorganischen Flüssigkeiten mit Hilfe eines Plasmaprozesses und/oder UV-Strahlung einer Wellenlänge unter 250 nm ohne die Verwendung von Metallen einer Ordnungszahl über 14 herstellbar ist mit einem atomaren Verhältnis von Sauerstoff zu Silizium von 0,75 bis 2,2 und einem atomaren Verhältnis von Kohlenstoff zu Silizium von 0,1 bis 2,5, jeweils gemessen mit XPS,
- als biokompatible Oberfläche,
- zum Vermitteln oder Versehen einer Oberfläche mit einer biokompatiblen Wirkung, und/oder
- zum Modifizieren einer biokompatiblen Wirkung einer Oberfläche.

Insbesondere kann eine biokompatible Wirkung erfindungsgemäß vermittelt oder modifiziert werden durch definierte Beeinflussung der chemischen Zusammensetzung der Oberfläche der siliziumhaltigen Schicht. Auf diese Weise kann die Adhäsion von Biomolekülen und/oder pro- und/oder eukaryontischen Zellen definiert eingestellt werden.

Unter einer Biokompatibilitätsschicht im Sinne der Erfindung wird daher insbesondere eine siliziumhaltige Schicht verstanden, enthaltend, bestehend im wesentlichen aus oder bestehend aus Silizium, O, C, H, optional N, die durch Plasmapolymerisation und/oder Vernetzung von siliziumorganischen Flüssigkeiten mit Hilfe eines Plasmaprozesses und/oder UV-Strahlung einer Wellenlänge unter 250 nm ohne die Verwendung von Metallen einer Ordnungszahl über 14 herstellbar ist.

Die Verwendung einer erfindungsgemäßen Biokompatibilitätsschicht erlaubt es insbesondere, die Biokompatibilität eines entsprechend beschichteten Gegenstandes wie eingangs beschrieben zu verbessern. Erfindungsgemäß zu verwendende Biokompatibilitätsschichten können leicht hergestellt werden, durch Verwendung geeigneter Precursoren können die Herstellungskosten gering gehalten werden, die Biokompatibilitätsschichten können sehr dünn und dennoch voll wirksam hergestellt werden, die Adhäsion von Zellen, Proteinen, Nukleinsäuren und Fettsäuren kann auf einfache Weise eingestellt werden. Die Herstellung entsprechender Biokompatibilitätsschichten kann als reiner Gasphasenprozess im Unterdruck durchgeführt werden, so dass eine Gefahr der Abgabe von nicht reagierten Monomeren praktisch vollständig vermieden werden kann. Erfindungsgemäße Biokompatibilitätsschichten können, wie eingangs beschrieben, auf einer Vielzahl unterschiedlicher fester Substrate aufgebracht werden, wobei auch kompliziert geformte Oberflächen gleichmäßig mit der Biokompatibilitätsschicht versehen werden können. Diese und andere Vorteile der erfindungsgemäß verwendeten Biokompatibilitätsschicht und entsprechend beschichteter Gegenstände werden nachfolgend näher dargestellt.

Durch die erfindungsgemäße Verwendung ist es reproduzierbar und mit überraschend geringem Aufwand möglich, für eine Vielzahl von Gegenständen und Oberflächen-Basismaterialien sowie für verschiedenste Anwendungsgebiete ein einheitliches Material zum Vermitteln biokompatibler Eigenschaften bereitzustellen. Die erfindungsgemäße Verwendung ermöglicht insbesondere, einen Gegenstand mit einer Oberfläche zu versehen, die gegenüber einem Menschen und seinen Organen
- keine oder lediglich akzeptable Zytotoxizität, gemessen gemäß ISO 10993-5:2003, insbesondere gemäß Punkt 8.3 der ISO 10993-5:2003, wobei die Kulturen in Kulturmedium mit 10 % Fötalem Kälberserum bei 37°C (± 2°C) bei 5% v/v Kohlendioxid inkubiert werden,
- keine oder lediglich akzeptable Reizungen oder intrakutane Reaktivität, gemessen gemäß ISO 10993-10:2003,
- keine oder lediglich akzeptable systemische Toxizität (akute Toxizität, subakute Toxizität und subchronische Toxizität), gemessen gemäß ISO 10993-11:2003,
- keine oder lediglich akzeptable Genotoxizität, gemessen gemäß ISO 10993-3:2003,
- ausreichende oder hohe Implantationsverträglichkeit, gemessen gemäß ISO 10993-6:2003, und/oder
- ausreichende oder hohe Hämokompatibilität, gemessen gemäß ISO 10993-4:2003,
aufweist, wobei je nach Einsatzgebiet des erfindungsgemäß beschichteten Gegenstandes eine oder mehrere der genannten Eigenschaften die Biokompatibilität des Gegenstandes begründen, dazu unten mehr.

Erfindungsgemäß wird daher ferner ein biokompatibler Gegenstand angegeben, umfassend einen Oberflächenbereich (Biokompatibilitäts-Oberfläche) mit einer vernetzten siliziumhaltigen Schicht mit einem atomaren Verhältnis von Sauerstoff zu Silizium von 0,75 bis 2,2, und einem atomaren Verhältnis von Kohlenstoff zu Silizium von 0,1 bis 2,5, gemessen mit XPS, wobei der Gegenstand ausgewählt ist aus der Gruppe bestehend aus:
a) einem bei bestimmungsgemäßem Gebrauch mit Körperflüssigkeit eines Lebewesens oder einem ihrer Bestandteile umströmten oder durchströmten Gegenstand,
b) einem bei bestimmungsgemäßem Gebrauch implantierten Gegenstand,
c) einem Behältnis zur Aufnahme und/oder zum Transport von Körperflüssigkeit, Gewebe oder deren Bestandteilen eines Lebewesens oder von Biomolekülen, bevorzugt Peptiden, Proteinen, Lipiden, Nukleinsäuren oder damit hergestellten Wirkstoffen,
d) einem Gegenstand zum zumindest teilweisen Bedecken von Haut oder Schleimhaut eines Lebewesens, und bevorzugt von Wunden,
e) einem bei bestimmungsgemäßem Gebrauch anderweitig mit Körperflüssig-keit, Gewebe oder deren Bestandteilen eines Lebewesens oder mit Biomolekülen, bevorzugt Peptiden, Proteinen, Nukleinsäuren oder damit hergestellten Wirkstoffen in Kontakt stehendem Gegenstand.

Erfindungsgemäße Biokompatibilitätsschichten werden also erfindungsgemäß eingesetzt, um auf Gegenständen insbesondere der Medizintechnik definierte Bedingungen bezüglich des Aufwachsens von Körperzellen einzustellen. Dabei kann sowohl ein gutes wie auch ein reduziertes Aufwachsen realisiert werden, wobei die jeweiligen Beschichtungen in beiden Anwendungsbereichen eine biokompatible Funktion aufzeigen. Parallel zum Aufwachsverhalten von Körperzellen kann die Bakterienadhäsion auf den beschichteten Oberflächen verringert werden. Hierbei konnte gezeigt werden, dass es erfindungsgemäße siliziumorganische Beschichtungen gibt, bei denen die Adhäsion von gramnegativen und auch gleichzeitig von grampositiven Bakterien stark vermindert wird. Blutkompatibilitätstests zeigen, dass keine Koagulation auftritt.

Ein bei bestimmungsgemäßem Gebrauch mit Körperflüssigkeit eines Lebewesens oder einem ihrer Bestandteile umströmter oder durchströmter Gegenstand im Sinne der Erfindung ist vorzugsweise eine Membran, ein Rohr oder ein Schlauch, insbesondere eine Oxygenatormembran, ein Katheter, ein Angioplasieballon, ein Stent oder eine Kanüle.

Ein bei bestimmungsgemäßem Gebrauch implantierter Gegenstand im Sinne der Erfindung ist vorzugsweise ein Implantat, ein medizinischer Nagel, eine Klammer, ein Faden und/oder eine Schraube, insbesondere ein Knochenfixationsnagel, ein Stent oder eine Gefäßprothese, ein Injektionssystem, ein Katheter, ein kardiovaskuläres Implantat, ein künstliches Organ, insbesondere ein Herzschrittmacher und eine Stromquelle desselben, eine Prothese, insbesondere ein künstlicher Gelenkkörper, insbesondere eine Gelenkpfanne und ein damit zusammenwirkendes Gegenstück, ein Cochlea-Implantat, eine künstliche Herzklappe, ein Herzklappenring oder eine Intraocularlinse.

Ein Behältnis zur Aufnahme und/oder zum Transport von Körperflüssigkeit, Gewebe oder deren Bestandteilen eines Lebewesens oder von Biomolekülen, bevorzugt Peptiden, Proteinen, Lipiden, Nukleinsäuren oder damit hergestellten Wirkstoffen im Sinne der Erfindung ist vorzugsweise eine Schale, ein Blutkonserven-Beutel, ein Zellkultur-Behältnis, ein Fermenter.

Ein Gegenstand zum zumindest teilweisen Bedecken von Haut oder Schleimhaut eines Lebewesens, und bevorzugt von Wunden, im Sinne der Erfindung ist vorzugsweise eine Wundauflage, ein Wundverband, ein Pflaster, eine Kontaktlinse, ein Inkontinenzprodukt.

Ein bei bestimmungsgemäßem Gebrauch anderweitig mit Körperflüssigkeit, Gewebe oder deren Bestandteilen eines Lebewesens oder mit Biomolekülen, bevorzugt Peptiden, Proteinen, Nukleinsäuren oder damit hergestellten Wirkstoffen in Kontakt stehender Gegenstand im Sinne der Erfindung ist vorzugsweise
- ein Sensor, insbesondere zur in-vivo-Analyse oder zur ex-vivo-Analyse, bevorzugt ein Sensor zur Untersuchung von Körperflüssigkeit, insbesondere Blut, Serum und Lymphe, Körperzellen, Gewebe, Organ(en),
- eine Sonde zum Einsatz im menschlichen oder tierischen Körper, bevorzugt eine Magensonde, ein Endoskop, eine Sonde zur Messung des Hirndrucks,
- eine Pumpe oder andere Vorrichtung zur Freisetzung von Substanzen im Körper
- ein Arzneimittelbehältnis, insbesondere enthaltend peptid-, protein-, fettsäure- und/oder nukleinsäurehaltige Inhalte, insbesondere Liposomen und/oder DNS-basierte Wirkstoffe oder Transportvehikel
- ein Teil eines Bioreaktors, auf dem Biokatalysatoren, wie zum Beispiel Enzyme oder Mikroorganismen, fixiert werden,
- ein Filtermaterial zum Herausfiltern von Zellen und/oder biologisch herstellbaren Makromolekülen aus einer Flüssigkeit.

Bevorzugt ist einerseits ein erfindungsgemäßer Gegenstand, dessen biokompatible Oberfläche ein atomares Verhältnis von Sauerstoff zu Silizium von 1,6 bis 2,2, ein atomares Verhältnis von Kohlenstoff zu Silizium von 0,1 bis 0,5 und ein atomares Verhältnis von Kohlenstoff zu Sauerstoff von 0,01 bis 0,2 aufweist. Derartige Schichten sind insbesondere hämokompatibel und dementsprechend besonders geeignet als Beschichtung von Oxygenatormembranen und anderen mit Blut in Berührung kommenden Oberflächen, insbesondere von Implantaten. Derartige Schichten besitzen einen Wasser-Kontaktwinkel von nicht mehr als 35°, vorzugsweise nicht mehr als 25° und besonders bevorzugt von 0-15°.

Bevorzugt ist andererseits auch ein erfindungsgemäßer Gegenstand, dessen biokompatible Oberfläche ein atomares Verhältnis von Sauerstoff zu Silizium von 0,75 bis 2,0, ein atomares Verhältnis von Kohlenstoff zu Silizium von 0,6 bis 2,5 und ein atomares Verhältnis von Kohlenstoff zu Sauerstoff von 0,4 bis 3,0 und ein atomares Verhältnis von Wasserstoff zu Kohlenstoff von 1,5 bis 3,2 aufweist.

An derartig beschichteten Gegenständen ist die Adhäsionsneigung biologischen Materials, insbesondere von Bakterien, Pilzen und eukaryontischen Zellen, stark herabgesetzt im Vergleich zu Metalloberflächen wie Titanoberflächen. Diese Oberflächen eignen sich daher besonders für solche erfindungsgemäßen Gegenstände, die frei von Bewuchs oder Anlagerung biologischen Materials bleiben sollen, wie Kanülen und Arzneimittelbehältnisse.

Besonders bevorzugt gelten für die Biokompatibilitätsschicht dieser Gegenstände die folgenden Stoffmengenverhältnisse:

| | | |
|---|---|---|
| 0,85 | < n(O) : n(Si) < | 1,8 |
| 0,8 | < n(C) : n(Si) < | 2,8 |
| 0,5 | < n(C) : n(O) < | 2,6 |
| 1,8 | < n(H) : n(C) < | 3,1. |

Ganz besonders bevorzugt gelten für die Biokompatibilitätsschicht dieser Gegenstände:

| | | |
|---|---|---|
| 1,0 | < n(O) : n(Si) < | 1,7 |
| 1,4 | < n(C) : n(Si) < | 2,6 |
| 0,9 | < n(C) : n(O) < | 2,4 |
| 2,2 | < n(H) : n(C) < | 3,0. |

Hierbei beziehen sich die Stoffmengenverhältnisse aller Atompaarungen ohne Wasserstoff auf XPS-Messungen bei Einstellungen, welche für ein als Standard eingesetztes trimethylsiloxy-terminiertes Polydimethylsiloxan (PDMS) mit einer kinematischen Viskosität von 350 mm²/s bei 25 °C und einer Dichte von 0,97 g/mL bei 25 °C Stoffmengenverhältnisse von n(O) : n(Si) = 1,02, n(C) : n(Si) = 2,35 und n(C) : n(O) = 2,29 ergeben. Das Verhältnis zwischen Wasserstoff und Kohlenstoff bezieht sich auf Ergebnisse der klassischen chemischen Elementaranalyse.

Hinsichtlich der Stoffmengenanteile der Elemente Silizium, Sauerstoff und Kohlenstoff gilt bevorzugt, dass die Biokompatibilitätsschicht dieser Gegenstände, bezogen auf 100 Atom-% für die Summe der Elemente Silizium, Sauerstoff und Kohlenstoff, enthält:

| | |
|---|---|
| Silizium: | 18 bis 30 Atom-% |
| Sauerstoff: | 20 bis 50 Atom-% |
| Kohlenstoff: | 25 bis 60 Atom-%. |

Besonders bevorzugt ist es aber, wenn die Biokompatibilitätsschicht dieser Gegenstände, bezogen auf 100 Atom-% für die Summe der Elemente Silizium, Sauerstoff und Kohlenstoff, enthält:

| | |
|---|---|
| Silizium: | 20 bis 28 Atom-% |
| Sauerstoff: | 22 bis 45 Atom-% |
| Kohlenstoff: | 35 bis 55 Atom-%. |

Hierbei beziehen sich die Atom-%-Angaben auf XPS-Messungen bei Einstellungen, welche für ein wiederum als Standard eingesetztes trimethylsiloxy-terminiertes Polydimethylsiloxan (PDMS) mit einer kinematischen Viskosität von 350 mm²/s bei 25 °C und einer Dichte von 0,97 g/mL bei 25 °C für Silizium 22,9 Atom-%, für Sauerstoff 23,4 Atom-% und für Kohlenstoff 53,75 Atom-% ergeben.

Unter Berücksichtigung bevorzugter Gewichtsanteile und Stoffmengenverhältnisse ist die Biokompatibilitätsschicht dieser Gegenstände besonders bevorzugt, wenn sie, bezogen auf 100 Atom-% für die Summe der Elemente Silizium, Sauerstoff und Kohlenstoff, enthält:

| | |
|---|---|
| Silizium: | 18 bis 30 Atom-% |
| Sauerstoff: | 20 bis 50 Atom-% |
| Kohlenstoff: | 25 bis 60 Atom-%, |

wobei für die Stoffmengenverhältnisse in der Biokompatibilitätsschicht dieser Gegenstände gilt:

| | | |
|---|---|---|
| 0,75 | < n(O) : n(Si) < | 2,0 |
| 0,6 | < n(C) : n(Si) < | 3,0 |
| 0,4 | < n(C) : n(O) < | 3,0 |
| 1,5 | < n(H) : n(C) < | 3,2 und |

wobei im XPS-Spektrum der Biokompatibilitätsschicht dieser Gegenstände im Vergleich mit einem trimethylsiloxy-terminierten Polydimethylsiloxan (PDMS) mit einer kinematischen Viskosität von 350 mm²/s bei 25 °C und einer Dichte von 0,97 g/mL bei 25 °C,
der Si 2p Peak einen Bindungsenergiewert besitzt, der um maximal 0,8 eV zu höheren oder niedrigeren Bindungsenergien verschoben ist und
der O 1s Peak einen Bindungsenergiewert besitzt, der um maximal 0,7 eV zu höheren oder niedrigeren Bindungsenergien verschoben ist.

Hinsichtlich der XPS-Messbedingungen und dem gewählten Standard gilt hierbei das oben Gesagte entsprechend.

Eine ganz besonders bevorzugte Biokompatibilitätsschicht dieser Gegenstände enthält, bezogen auf 100 Atom-% für die Summe der Elemente Silizium, Sauerstoff und Kohlenstoff:

| | |
|---|---|
| Silizium: | 20 bis 28 Atom-% |
| Sauerstoff: | 22 bis 32 Atom-% |
| Kohlenstoff: | 38 bis 53 Atom-%, |

wobei für die Stoffmengenverhältnisse der Biokompatibilitätsschicht dieser Gegenstände gilt:

| | | |
|---|---|---|
| 1,0 | < n(O) : n(Si) < | 1,7 |
| 1,4 | < n(C) : n(Si) < | 2,6 |
| 0,9 | < n(C) : n(O) < | 2,4 |
| 2,2 | < n(H) : n(C) < | 3,0 und |

wobei im besagten ESCA-Spektrum der Si 2p Peak einen Bindungsenergiewert besitzt, der um maximal 0,60 eV zu höheren oder niedrigeren Bindungsenergien verschoben ist und der O 1s Peak einen Bindungsenergiewert besitzt, der um maximal 0,65 eV zu höheren oder niedrigeren Bindungsenergien verschoben ist.

Zudem weist die erfindungsgemäße Biokompatibilitätsschicht dieser Gegenstände beim Messen mit fortschreitendem Kontaktwinkel auf einer ebenen Oberfläche bei 25°C einen Wasser-Kontaktwinkel von zumindest 90°, vorzugsweise zumindest 95° und besonders bevorzugt zumindest 100° auf.

Insbesondere bevorzugt ist ein erfindungsgemäßer Gegenstand mit einer Biokompatibilitätsschicht, die zu mehr als 98% aus den Elementen Silizium, Kohlenstoff, Sauerstoff, Wasserstoff und Stickstoff besteht. Weiter bevorzugt ist ein erfindungsgemäßer Gegenstand mit einer Biokompatibilitätsschicht, die zu mehr als 98% aus den Elementen Silizium, Kohlenstoff, Sauerstoff und Wasserstoff besteht. Solche Schichten lassen sich, wie nachfolgend noch weiter beschrieben wird, besonders gut zum Verwirklichen der oben genannten Vorteile herstellen.

In einer speziellen Ausgestaltung der Erfindung beträgt der Kohlenstoff-Anteil in der Biokompatibilitätsschicht 3-15%. Hierbei weisen die Kohlenstoffatome der Biokompatibilitätsschicht vorzugsweise einen Anteil von 5-35% an Kohlenstoffatomen mit einer Bindung zu genau einem Sauerstoff-Atom ("C-O") auf, gemessen mit XPS. Entsprechend liegen 5-35% der Kohlenstoffatome der siliziumhaltigen Schicht als Hydroxyl- oder Ethergruppen vor. Stärker bevorzugt ist ein Anteil von 10-30%, vorzugsweise von 15-25%. Auf diese Weise lassen sich besonders hydrophile und hämokompatible Schichten herstellen.

Ebenfalls bevorzugt ist eine Biokompatibilitätsschicht, die einen Anteil von 5-20% an Kohlenstoffatomen mit einer Bindung zu genau zwei Sauerstoff-Atomen ("COO") aufweist, gemessen mit XPS. Entsprechend liegen 5-20% der Kohlenstoffatome der siliziumhaltigen Schicht als Ester- oder Säuregruppen vor. Stärker bevorzugt ist ein Anteil von 5-10%. Diese polaren Gruppen fördern ebenfalls die Hydrophilie der erfindungsgemäßen Biokompatibilitätsschicht.

Der Anteil an Kohlenstoffatomen mit einer Bindung zu genau einem Sauerstoff-Atom ("C-O") sowie zu genau zwei Sauerstoff-Atomen ("COO") wird ermittelt, indem die XPS-Spektren, welche zur Probennormalen orientiert gemessen worden sind, einer Kurvenanpassung (einem Curve-Fitting) unterzogen werden. Unter der Vorraussetzung, dass die Stickstoffkonzentration in der Beschichtung klein ist (< 0.5 Atom-%) werden die chemischen Verschiebungen (Shifts) beim C1s-Spektrum wie in Tabelle 1 angegeben interpretiert:

**Tabelle 1**

| Abkürzung | Spektrennummer | Gruppen | Bindungsenergie |
|---|---|---|---|
| "C" | I | Aliphatisch | 285,0 eV |
| "CO" | II | Alkohol, Ether | 286.5 ± 0.2 eV |
| "COO" | III | Carboxyl, Ester | 289.2 ± 0.2 eV |

Der aliphatische Kohlenstoff wird per Definition auf 285,0 eV gesetzt. Zur Vorbereitung des Curve-Fittings wird eine Baseline-Korrektur des C1s-Spektrums nach Shirley im Bereich von 281 eV bis 292 eV durchgeführt. Anschließend wird mit Hilfe von Gauß-Lorentz-Funktionen gefittet. Hierzu wird das Maximum der Bindungsenergie für den aliphatischen Kohlenstoff auf 285.0 eV, das Maximum des "C-O"-Kohlenstoffs auf 286.5 eV bzw. auf 289.2 eV für den "COO"-Kohlenstoff gelegt. Als Fitparameter wird die Energie im Bereich von ± 0.2 eV, die Zählrate und die Halbwertsbreite angesetzt. Die Bindungsenergie des aliphatischen Kohlenstoffs wird nicht mit angefittet. Der Fit ist dann beendet, wenn der Least-Square-Fit-Algorithmus sein Minimum annimmt. Aus den Flächenverhältnissen der Funktion für den aliphatischen Kohlenstoff bzw. den Funktionen für "CO"- und "COO"-Kohlenstoff lassen sich die gruppenspezifischen Konzentrationen berechnen. Weiterhin wird bei der Quantifizierung der Konzentrationen angenommen, dass sich die funktionellen Gruppen in der gesamten Informationstiefe des XPS-Spektrums homogen verteilen, so dass die gemessene Beschichtung mindestens 10 nm dick sein muss.

Die Biokompatibilitätsschicht ist hergestellt oder herstellbar durch Plasmapolymerisation eines Methylsiloxan-Precursors, vorzugsweise von Hexamethyldisiloxan, insbesondere durch Niederdruck- oder Atmosphärendruck-Plasmapolymerisation, oder durch Vernetzen eines Silikonöls ohne chemisch reaktive Gruppen unter Einwirkung eines Plasmas oder UV-Strahlung einer Wellenlänge unter 250 nm, insbesondere Excimer-Strahlung.

Eine "plasmapolymere Schicht" ist im Rahmen dieses Textes eine Schicht, welche mittels Plasmapolymerisation herstellbar ist. Plasmapolymerisation ist ein Verfahren, bei dem sich gasförmige Precursoren (oft auch Monomere genannt), angeregt durch ein Plasma, auf einem frei wählbaren Substrat als hochvernetzte Schicht niederschlagen. Voraussetzung für eine Plasmapolymerisation ist das Vorhandensein von kettenbildenden Atomen wie Kohlenstoff oder Silizium im Arbeitsgas. Durch die Anregung werden die Moleküle der gasförmigen Substanz (Precursoren), durch den Beschuss mit Elektronen und/oder energiereichen Ionen fragmentiert. Dabei entstehen hochangeregte radikalische oder ionische Molekülfragmente, die miteinander im Gasraum reagieren und auf der zu beschichtenden Oberfläche abgeschieden werden. Auf diese abgeschiedene Schicht wirkt die elektrische Entladung des Plasmas und dessen intensiver lonen- und Elektronenbeschuss fortwährend ein, so dass in der abgeschiedenen Schicht weitere Reaktionen ausgelöst und eine hochgradige Verknüpfung (Vernetzung) der abgeschiedenen Moleküle erzielt werden kann.

Auch Kombinationen der Beschichtungsverfahren sind möglich, beispielsweise die Plasmavernetzung von Siliconöl kombiniert mit einer Plasmabeschichtung.

Als Flüssigkeitsfilm wird im letzteren Fall auf die Oberfläche (z. B. des Trägers) zunächst eine Substanz aufgebracht, die kettenbildende Atome wie Kohlenstoff oder Silizium beinhaltet, z.B. Silikonöl. Wird der Flüssigkeitsfilm einem Plasma ausgesetzt, so wirken die Elektronen und/oder energiereichen Ionen und die im Plasma erzeugte UV-Strahlung auf die Flüssigkeitsmoleküle ein. In der Flüssigkeit entstehen Bindungsbrüche, die zu einer Vernetzung der Flüssigkeitsmoleküle führen. Über die Expositionsdosis kann der Fachmann einen geeigneten Vernetzungsgrad des Flüssigkeitsfilms erzielen.

Im Rahmen des vorliegenden Textes umfasst der Begriff "plasmapolymere Schicht" auch Schichten, die mittels plasmaunterstützter CVD (PE-CVD) hergestellt werden können.

Ferner sei ausdrücklich erwähnt, dass auch Atmosphärendruckplasmaverfahren zur Herstellung erfindungsgemäß einzusetzender plasmapolymerer Schichten verwendet werden können, wenngleich Niederdruck-Plasmapolymerisationsverfahren derzeit bevorzugt sind.

Im Rahmen des vorliegenden Textes werden Substanzen, die zur Schichtbildung über eine Plasmapolymerisation als Gas bzw. Dampf einem Plasma zugeführt werden, als "Monomere" (gasförmige Precursoren) bezeichnet. Als "flüssige Precursoren" werden Flüssigkeiten bezeichnet, welche beispielsweise durch die Einwirkung eines Plasmas vernetzt werden können (beispielsweise durch hochangeregte Teilchen, Elektronen oder UV-Strahlung), ohne vorher zu verdampfen.

Plasmapolymere Schichten sind in ihrer chemischen und strukturellen Zusammensetzung eindeutig von polymeren Schichten zu unterscheiden. Während bei Polymeren der Verknüpfungsprozess der Monomere in vorhersagbarer Weise geschieht, werden bei der Plasmapolymerisation die eingesetzten Monomere fragmentiert (bis zur vollständigen Zerstörung) und in Form von reaktiven Spezies abgeschieden, so dass sich eine vernetzte Schicht ergibt, ohne Bereiche mit regelmäßigen Wiederholungseinheiten. Diese entstehende Schicht ist zusätzlich noch dem Plasma ausgesetzt, so dass sie durch Ablation und Redepositionseffekte weiter modifiziert wird. Die entstehende Schicht ist dreidimensional vernetzt und amorph. Dementsprechend unterscheidet sich die Plasmapolymerisation im Sinne dieses Textes von konventionellen Methoden der Polymerisation. Dies gilt auch für die sogenannte "strukturerhaltende Plasmapolymerisation", da selbst bei "milden" Plasmabedingungen unvorhersagbare Molekülbrüche auftreten. In diesem Zusammenhang sei beispielsweise auf folgende Literaturstelle verwiesen: "Plasma Polymerization" by H. Yasuda, Academic Press, Inc., (1985)

Nach der Herstellung einer erfindungsgemäßen Biokompatibilitätsschicht durch das Vernetzen des Precursors bzw. Silikonöls kann diese zumindest teilweise oxidiert werden, vorzugsweise durch Plasma-Einwirkung ("Aktivierung"), Beflammung, Oxy-Fluorierung, Laser-Behandlung oder Behandlung mit Excimer-Lampen. Insbesondere durch Oxidation kann die chemische Zusammensetzung der Oberfläche der Beschichtung auf einfache Weise definiert eingestellt werden. Auf diese Weise ist es beispielsweise möglich, die oben aufgezählten verschiedenen erfindungsgemäßen Gegenstände mit einer hydrophilen Schicht zu versehen, insbesondere mit einer Schicht, auf der sich direkt nach der Herstellung ein Wasserkontaktwinkel von nicht mehr als 35° einstellt, um die oben aufgezählten Vorteile zu erreichen.

Durch das Einstellen der chemischen Oberflächenzusammensetzung ist insbesondere möglich,
- Fördern eines Anhaftens von Gewebezellen und/oder
- Reduzieren einer Anhaftung von Gewebezellen und/oder
- Reduzieren einer Anhaftung von Gewebezellen auf einem Teil der Oberfläche des biokompatiblen Gegenstands und Fördern der Anhaftung auf einem anderen Teil der Oberfläche des biokompatiblen Gegenstands und/oder
- Reduzieren einer Anhaftung pathogener Ablagerungen und/oder
- Reduzieren einer Anhaftung von Bakterien und/oder
- Reduzieren der Thrombogenität und/oder
- Reduzieren des Auftretens humoraler und zellulärer Immunreaktionen und/oder
- Reduzieren von unspezifischer Adsorption von Peptiden, Proteinen, Lipiden und/oder Nukleinsäuren an der Oberfläche des biokompatiblen Gegenstands und/oder
- Ermöglichen räumlich begrenzten Wachstums von Zellkulturen auf der Oberfläche des biokompatiblen Gegenstands und/oder,
- Erhöhen der Benetzbarkeit durch wässrige Flüssigkeiten, insbesondere Blut.

Zum Begrenzen des zu oxidierenden Bereichs der Biokompatibilitätsschicht wird vorzugsweise eine stoffschlüssig aufliegende Maske, vorzugsweise eine ablösbare selbstklebende Maske, besonders bevorzugt ein Klebeband, und/oder ein, vorzugsweise in Wasser, zumindest teilweise löslicher Stoff oder eine gedruckte Maske verwendet und vorzugsweise nach dem Oxidieren entfernt. Auf diese Weise ist eine besonders einfache Begrenzung des oxidierten Bereiches möglich.

Durch einfache Maskierungen oder z.B. lokale Laserbehandlung können auch sowohl wachstumsfördernde als auch wachstumshemmende Oberflächenbereiche auf ein und demselben Bauteil realisiert werden.

Die Biokompatibilitätsschicht besitzt vorzugsweise eine Schichtdicke von nicht mehr als 2 µm, bevorzugt nicht mehr als 1 µm, ferner bevorzugt nicht mehr als 500 nm, und dabei jeweils von zumindest 5 nm, vorzugsweise zumindest 10 nm und besonders bevorzugt von zumindest 15 nm.

Erfindungsgemäß wird insbesondere auch ein biokompatibilitätsbeschichteter Gegenstand angegeben, umfassend eine Biokompatibilitätsschicht wie zuvor beschrieben. Die Biokompatibilitätsschicht ist dabei vorzugsweise angeordnet auf einer Außen- und/oder Innen-Oberfläche des beschriebenen erfindungsgemäßen Gegenstandes. Zweckmäßigerweise wird die Biokompatibilitätsschicht auf derjenigen Oberfläche angeordnet, die bei bestimmungsgemäßem Gebrauch des Gegenstandes mit dem zu schützenden Gut in Kontakt kommt, also beispielsweise die Innenseite einer Spritze bzw. die Außenseite eines Knochenfixationsnagels.

Erfindungsgemäß wird ferner die Verwendung einer Biokompatibilitätsschicht angegeben zum Beeinflussen, Modifizieren oder Ändern der Zytotoxizität, gemessen gemäß ISO 10993-5:2003, der Reizungen oder intrakutaner Reaktivität, gemessen gemäß ISO 10993-10:2003, der systemischen Toxizität (akute Toxizität, subakute Toxizität und subchronische Toxizität), gemessen gemäß ISO 10993-11:2003, der Genotoxizität, gemessen gemäß ISO 10993-3:2003, der Implantationsverträglichkeit, gemessen gemäß ISO 10993-6:2003, und/oder der Hämokompatibilität, gemessen gemäß ISO 10993-4:2003 eines Gegenstandes.

Aufgabe der vorliegenden Erfindung war es, Mittel anzugeben, mit denen die Übertragung von Funktionsschichten auf gegebenenfalls entstehende Substratoberflächen sauber, mit einer guten Beschichtungsqualität und in bereits bekannte Arbeitsprozesse gut integrierbar bewerkstelligt werden kann.

Die Erfindung wird nachfolgend anhand der Beispiele und der Figuren näher beschrieben, ohne dass dadurch der Schutzbereich der Ansprüche eingeschränkt werden soll.

Die beigefügten Figuren 1 bis 16 zeigen:
- Figur 1:: XPS-Übersichtsspektrum der Beschichtung Plasmapolymer A1
- Figur 2:: XPS-Detailspektrum des O 1s Peaks der Beschichtung Plasmapolymer A1
- Figur 3:: XPS-Detailspektrum des C 1s Peaks der Beschichtung Plasmapolymer A1
- Figur 4:: XPS-Detailspektrum des Si 2p Peaks der Beschichtung Plasmapolymer A1
- Figur 5:: C1s-Spektrum der Beschichtung Plasmapolymer C1
- Figur 6:: C1s-Spektrum der Beschichtung Plasmapolymer C2
- Figur 7:: C1s-Spektrum der Beschichtung Plasmapolymer C3
- Figur 8:: C1s-Spektrum der Beschichtung Plasmapolymer C4
- Figur 9:: Zellwachstumsmanagement durch Biokompatibilitätsschichten auf Aluminium
- Figur 10:: Zellwachstumsmanagement durch Biokompatibilitätsschichten auf Edelstahl
- Figur 11:: Zellwachstumsmanagement durch Biokompatibilitätsschichten auf Glas
- Figur 12:: Zellwachstumsmanagement durch Biokompatibilitätsschichten unter Zuhilfenahme von Excimer-Lampen auf Glas
- Figur 13:: Zellwachstumsmanagement durch Biokompatibilitätsschichten auf Keramik
- Figur 14:: Zellwachstumsmanagement durch Biokompatibilitätsschichten auf PMMA
- Figur 15:: Zellwachstumsmanagement durch Biokompatibilitätsschichten auf Silikon
- Figur 16:: Zellwachstumsmanagement durch Biokompatibilitätsschichten auf Titan

### Beispiele

### Beispiel 1: Plasmapolymerbeschichtungen aus einem 1 m³ Reaktor

Flache Substrate der nachfolgend angeführten Werkstoffe wurden mittels eines Niederdruck-Plasmaverfahrens in einem 1m³ - Reaktor (Beschreibung siehe ISBN 978-3-86727-548-4 "Aufskalierung plasmapolymerer Beschichtungsverfahren", Seite 21 - 26 von Dr. Klaus Vissing) mit einer erfindungsgemäßen Schicht versehen:
- Aluminium als Abschnitte (ca. 10 x 10 mm)
- Edelstahl als Abschnitte (ca. 10 x 10 mm)
- Glas als Abschnitte (ca. 10 x 10 mm)
- Aluminiumoxid-Keramik Al23 (von Degussit- Friatec)
- Polymethylmethacrylat (PMMA) als Abschnitte (ca. 10 x 10 mm) aus Sterilverpackungsflaschen
- Silikon als Abschnitte (ca. 15*10 mm)
- Titan als Abschnitte (ca. 15*10 mm)

Hierfür wurden die Proben nach dem Stand der Technik im Plasma mit Sauerstoff feingereinigt sowie je nach Bedarf aktiviert und/oder mit einer Haftvermittlungsschicht versehen. Anschließend wurde bei einer Frequenz von 13,56 MHz die erfindungsgemäße plasmapolymere Beschichtung appliziert, wobei dem Plasma O₂ und Hexamethyldisiloxan (HMDSO) zugeführt wurden. Die genauen Verfahrensparameter für die Abscheidung der plasmapolymeren Beschichtung sind in Tabelle 2 angegebenen. Die Zeitspanne für die plasmapolymere Beschichtung variierte von 9 bis 60 Minuten. Hierbei wurden Schichten zwischen 90 und 250 nm Dicke aufgebracht.

**Tabelle 2**

| **Beschichtung** | **Gasfluss HMDSO (Sccm)** | **Gasfluss O₂ (Sccm)** | **Gasfluss H₂ (Sccm)** | **Druck (mbar)** | **Leistung (W)** |
|---|---|---|---|---|---|
| Plasmapolymer A1 | 67 | 20 | | 0,025 | 700 |
| Plasmapolymer A2 | 27 | 20 | 200 | 0,025 | 1600 |
| Plasmapolymer A3 | 27 | 100 | | 0,023 | 2500 |

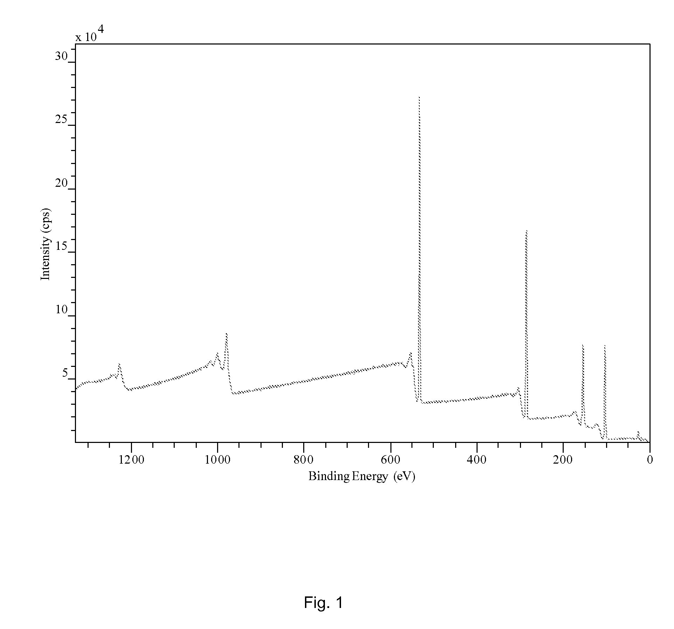

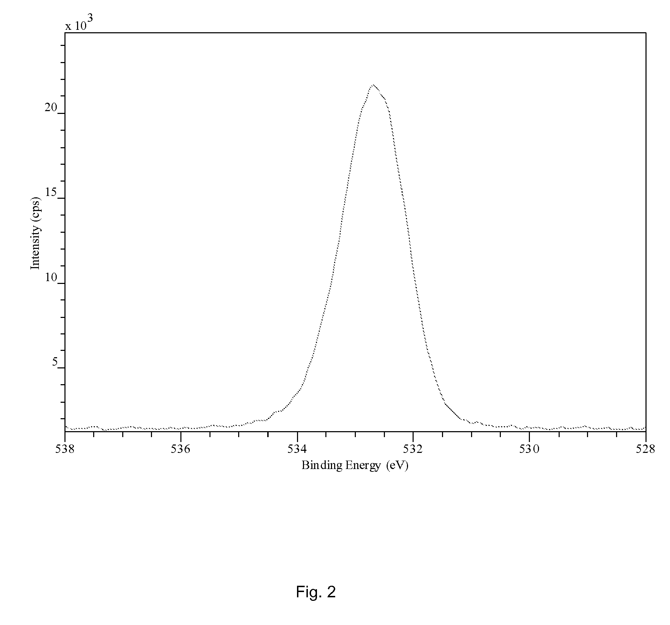

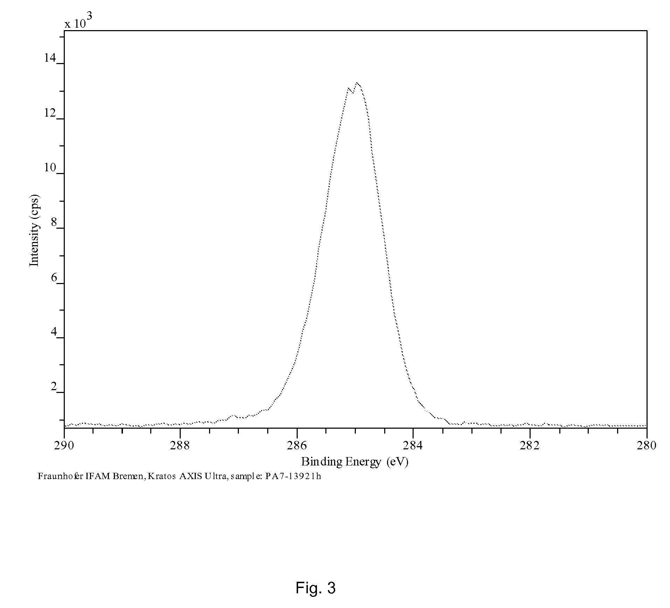

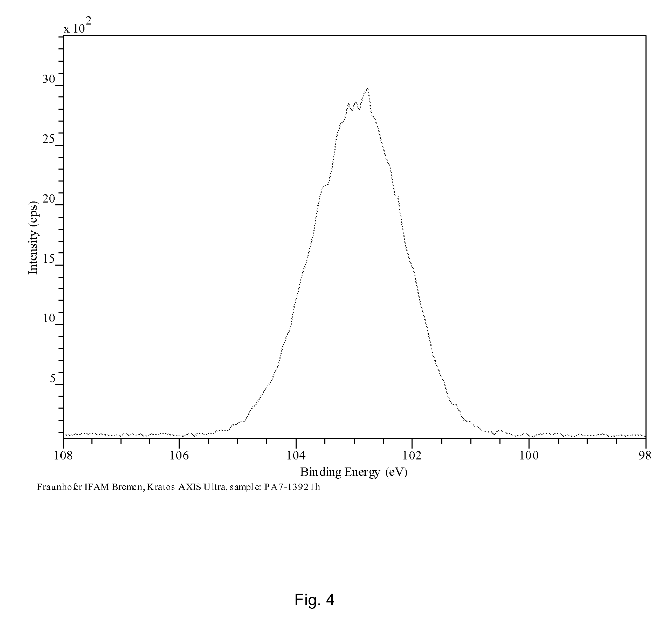

In den Figuren 1 bis 4 sind XPS-Spektren der Beschichtung Plasmapolymer A1 dargestellt.

Zudem wurden analog zum beschriebenen Verfahren für die Plasmapolymere A1 bis A3 plasmapolymere Beschichtungen unter Bedingungen wie in Tabelle 3 beschrieben hergestellt und dabei in einem weiteren Prozessschritt 60 Sekunden im NiederdruckPlasma aktiviert. Für diese Schichten, die eine verbesserte Anhaftung von Biomolekülen und Zellen ermöglichten, sind die Prozessparameter für die plasmapolymere Beschichtung und die Aktivierung in Tabelle 3 angegeben. Die Zeitspanne für die plasmapolymere Beschichtung variierte von 12 bis 19 Minuten. Hierbei wurden Schichten zwischen 50 und 170 nm aufgebracht.

**Tabelle 3**

| | **Beschichtungsprozessschritt** | | | | **Aktivierungsprozessschritt** | | |
|---|---|---|---|---|---|---|---|
| | **Gasfluss** | | **Druck** **(mbar)** | **Leistung** **(W)** | **Gasfluss O₂** **(Sccm)** | **Druck** **(mbar)** | **Leistung** **(W)** |
| **Beschichtung** | **HMDSO** **(Sccm)** | **O₂** **(Sccm)** | | | | | |
| Plasmapolymer B1 | 13 | 500 | 0,025 | 2000 | 500 | 0,025 | 2000 |
| Plasmapolymer B2 | 10 | 500 | 0,03 | 2000 | 500 | 0,03 | 2000 |
| Plasmapolymer B3 | 10 | 500 | 0,03 | 2000 | 500 | 0,025 | 2000 |

### Erläuterungen zu den XPS-Messunqen

Die XPS-Untersuchungen wurden mit dem Spektrometer KRATOS AXIS Ultra der Fa. Kratos Analytical durchgeführt. Die Analysekammer war mit einer Röntgenquelle für monochromatisierte Al K_{α} - Strahlung, einer Elektronenquelle als Neutralisator und einem Quadrupolmassenspektrometer ausgerüstet. Weiterhin verfügte die Anlage über eine magnetische Linse, welche die Photoelektronen über einen Eintrittsschlitz in einen Halbkugelanalysator fokussierte. Durch Kalibrierung wurde der aliphatische Anteil des C 1s Peaks auf 285,0 eV gesetzt. Während der Messung zeigte die Oberflächennormale auf den Eintrittsschlitz des Halbkugelanalysators.

Die Passenergie betrug bei der Bestimmung der Stoffmengenverhältnisse jeweils 160 eV, die entsprechenden Spektren werden als Übersichtsspektren bezeichnet. Bei der Bestimmung der Peak-Parameter betrug die Passenergie jeweils 20 eV.

Die genannten Messbedingungen sind bevorzugt, um eine weitgehende Unabhängigkeit vom Spektrometertyp zu ermöglichen und um erfindungsgemäße plasmapolymere Produkte zu identifizieren.

Als Referenzmaterial wurde das Polydimethylsiloxan, Silikonöl DMS-T23E der Firma Gelest Inc. (Morrisville, USA) verwendet. Dieses trimethylsiloxy-terminierte Silikonöl besitzt eine kinematische Viskosität von 350 mm²/s (±10%) und eine Dichte von 0,970 g/ml bei 25 °C sowie ein mittleres Molekulargewicht von ca. 13.650 g/mol. Das ausgewählte Material zeichnet sich durch einen extrem geringen Anteil an verdampfbaren Bestandteilen aus: nach 24 Stunden bei 125 °C und 10⁻⁵ Torr wurden weniger als 0,01% flüchtige Anteile nachgewiesen (nach ASTM-E595-85 und NASA SP-R0022A). Es wurde mit Hilfe eines Spin-Coating-Prozesses als 40 bzw. 50 nm dicke Schicht auf einen Siliziumwafer aufgetragen; dabei wurde als Lösemittel Hexamethyldisiloxan verwendet.

### Beispiel 2: Plasmapolymerbeschichtungen aus einem 250 I Reaktor

Die plasmapolymeren Beschichtungen C1 bis C6 (siehe unten) wurden mit einem Niederdruck-Plasmareaktor, der ein Volumen von 250 I (Beschreibung in ISBN 3-8265-9216-6 "Charakterisierung der spektroskopischen Eigenschaften von Metall- und Halbleiterclustern in plasmapolymeren Matrizen" von Dr. Dirk Salz) aufweist, durchgeführt. Die elektrische Anregungsspannung hat eine Frequenz von 13,56 MHz. Der Abstand zwischen der Plasmaentladungselektrode und dem Substrat (SiliziumWafer) betrug 30 cm. Vor jedem Experiment wurde der Niederdruck-Reaktor auf einen Druck von 0.01 mbar evakuiert. Das Restgas bei diesem Druck besteht zu mehr als 95% aus Wasserdampf. Die Beschichtungen wurden mit XPS untersucht. Die C1s-Spektren wurden einem Curve-Fitting unterzogen. Zudem wurde innerhalb von weniger als 12 Stunden auf den beschichteten Siliziumwafern der Kontaktwinkel von Wasser mit fortschreitendem Tropfen bei 25°C gemessen.

### Ausführungsbeispiel Plasmapolymer C1

Zwei Siliziumwafer wurden in drei Prozessschritten beschichtet. Im ersten Schritt wurde eine Sauerstofffeinreinigung durchgeführt, anschließend der Beschichtungsschritt mit Hexamethyldisiloxan und abschließend eine Sauerstoffaktivierung. Die genauen Beschichtungsparameter sind in Tabelle 4 angegeben. Es wurden Schichten mit einer Dicke von ca. 60 nm hergestellt. Der Kontaktwinkel von Wasser betrug 23°.

**Tabelle 4: Beschichtungsparameter des Ausführungsbeispiels Plasmapolymer C1**

| | Feinreinigung | Beschichtung | Aktivierung |
|---|---|---|---|
| O₂-Fluss / sccm | 60 | 60 | 60 |
| HMDSO-Fluss / sccm | 0 | 5 | 0 |
| Plasmaleistung / W | 800 | 800 | 800 |
| Behandlungszeit / min | 10 | 10 | 10 |
| Prozessdruck / mbar | 0,022 | 0,026 | 0,022 |

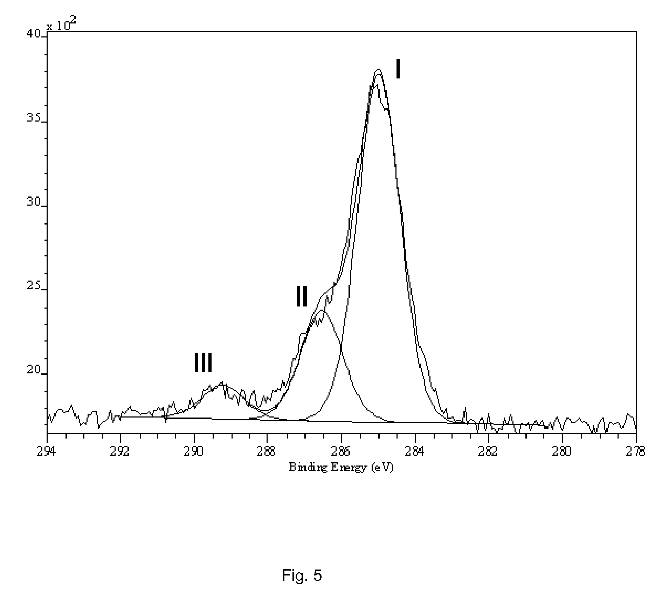

Die XPS-Analyse liefert die atomare Zusammensetzung Sauerstoff = 65,9%, Silizium = 27,0%, Kohlenstoff = 7,03%, Stickstoff = 0,12%. Das experimentell bestimmte hoch aufgelöste C1s-Spektrum zeigt Figur 5 als Einhüllende der drei einzelnen Gauß-Lorentz-Funktionen, die mit einem Curve-Fit bestimmt worden sind. Die Flächenquantifizierung ergibt folgendes Ergebnis für den Kohlenstoff: Aliphatischer Kohlenstoffanteil = 77,1% (285 eV), CO-Gruppen = 16,5% (286,6 eV) und COO-Gruppen = 6,4% (289,3 eV). Die Zahlen in Klammern entsprechen dem energetischen Maximum der Gauß-Lorentz-Funktionen.

### Ausführungsbeispiel Plasmapolymer C2

Dieses Experiment wurde wie Ausführungsbeispiel C1 durchgeführt, jedoch betrug die Aktivierungszeit 20 min, statt 10 min.

**Tabelle 5: Beschichtungsparameter des Ausführungsbeispiels Plasmapolymer C2**

| | Feinreinigung | Beschichtung | Aktivierung |
|---|---|---|---|
| O₂-Fluss / sccm | 60 | 60 | 60 |
| HMDSO-Fluss / sccm | 0 | 5 | 0 |
| Plasmaleistung / W | 800 | 800 | 800 |
| Behandlungszeit / min | 10 | 10 | 20 |
| Prozessdruck / mbar | 0,022 | 0,026 | 0,022 |

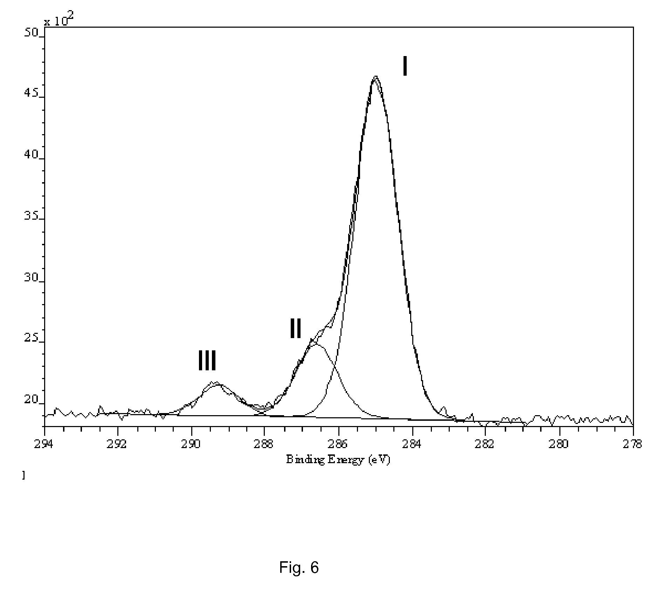

Die Schichtdicke und der Wasserkontaktwinkel auf den Si-Wafern betragen 60 nm bzw. 13°. Die XPS-Analyse liefert folgende atomare Zusammensetzung: Sauerstoff = 66,2%, Silizium = 27,4%, Kohlenstoff = 6,29%, Stickstoff = 0,13%. Das Ergebnis des Curve-Fits (Figur 6) liefert folgende Konzentrationen für den Kohlenstoff: Aliphatischer Kohlenstoffanteil = 76,8 % (285 eV), CO-Gruppen = 16,4% (286,5 eV) und COO-Gruppen = 6,8% (289,2 eV).

### Ausführungsbeispiel Plasmapolymer C3

Dieses Experiment wurde wie Ausführungsbeispiel C1 durchgeführt, jedoch fand die Aktivierung mit Wasserdampf statt, welcher ständig aus den Reaktorwänden desorbiert wird.

**Tabelle 6: Beschichtungsparameter des Ausführungsbeispiels Plasmapolymer C3**

| | Feinreinigung | Beschichtung | Wasserdampfplasma |
|---|---|---|---|
| O₂-Fluss / sccm | 60 | 60 | 0 |
| HMDSO-Fluss / sccm | 0 | 5 | 0 |
| Plasmaleistung / W | 800 | 800 | 800 |
| Behandlungszeit / min | 10 | 10 | 10 |
| Prozessdruck / mbar | 0,022 | 0,026 | 0,022 |

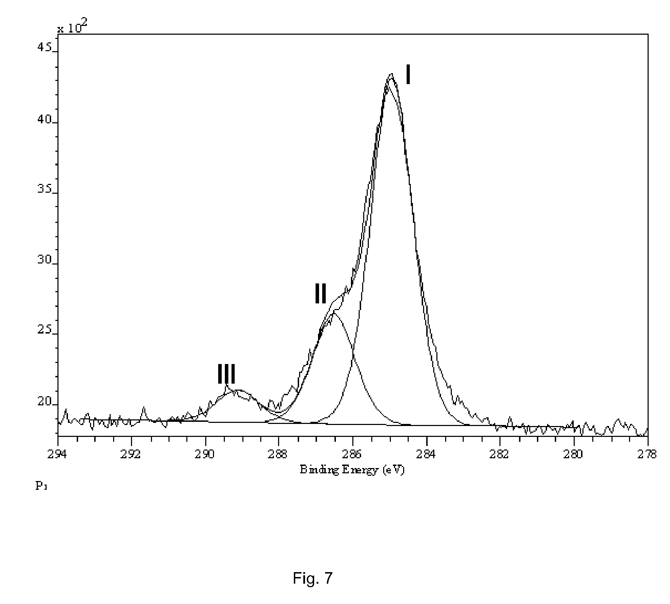

Die Schichtdicke und der Wasserkontaktwinkel auf den Si-Wafern betragen 60 nm bzw. 5°. Die XPS-Analyse liefert für die ersten 10 nm folgende atomare Zusammensetzung: Sauerstoff = 65,7%, Silizium = 27,2%, Kohlenstoff = 6,96%, Stickstoff = 0,12%. Das Ergebnis des Curve-Fits (Figur 7) liefert folgende Konzentrationen für den Kohlenstoff: Aliphatischer Kohlenstoffanteil = 73,4% (285 eV), CO-Gruppen = 21,2% (286,5 eV) und COO-Gruppen = 5,4 % (289,1 eV).

### Ausführungsbeispiel C4

Dieses Experiment wurde wie Ausführungsbeispiel C2 durchgeführt, jedoch fand die Aktivierung mit Wasserdampf 20 min lang statt, welcher ständig aus den Reaktorwänden desorbiert wird.

**Tabelle 7: Beschichtungsparameter des Ausführungsbeispiels Plasmapolymer C4**

| | Feinreinigung | Beschichtung | Wasserdampfplasma |
|---|---|---|---|
| O₂-Fluss / sccm | 60 | 60 | 0 |
| HMDSO-Fluss / sccm | 0 | 5 | 0 |
| Plasmaleistung / W | 800 | 800 | 800 |
| Behandlungszeit / min | 10 | 10 | 20 |
| Prozessdruck / mbar | 0,022 | 0,026 | 0,022 |

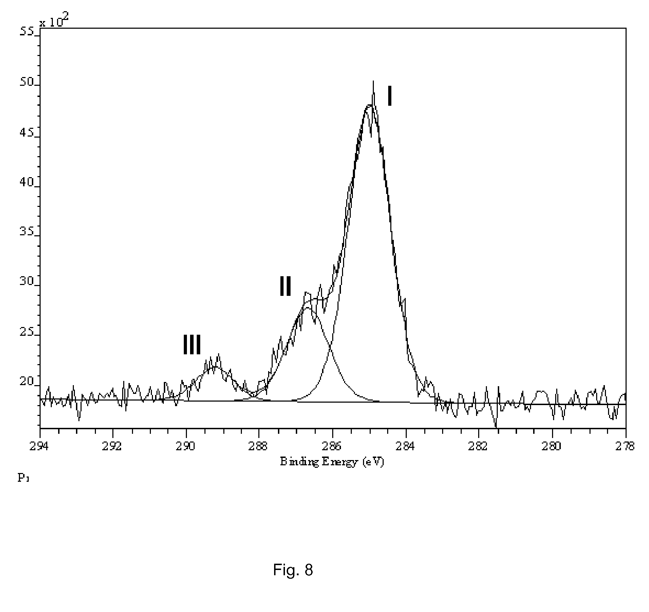

Die Schichtdicke und der Wasserkontaktwinkel auf den Si-Wafern betragen 60 nm bzw. annähernd 0 Grad. Die XPS-Analyse liefert für die ersten 10 nm folgende atomare Zusammensetzung: Sauerstoff = 65,5%, Silizium = 26,9%, Kohlenstoff = 7,44%, Stickstoff = 0,14%. Das Ergebnis des Curve-Fits (Figur 8) liefert folgende Konzentrationen für den Kohlenstoff: Aliphatischer Kohlenstoffanteil = 70,4 % (285 eV), CO-Gruppen = 22,4 % (286,7 eV) und COO-Gruppen = 7.1 % (289,2 eV).

### Ausführungsbeispiel Plasmapolymer C5

Für dieses Beispiel wurde Polyurethan (PU) mit einer hydrophilen ca. 40 nm dicken Plasmabeschichtung versehen. Der Wasserkontaktwinkel beträgt annähernd 0°. Die Beschichtungsparameter zeigt Tabelle 8 Die Bakterienadhäsion wurde mit dem Adhäsionsassay nach ISO 17025 bestimmt.

**Tabelle 8: Beschichtungsparameter des Ausführungsbeispiels Plasmapolymer C5**

| | Feinreinigung | Beschichtung | Aktivierung |
|---|---|---|---|
| O₂-Fluss / sccm | 60 | 400 | 100 |
| HMDSO-Fluss / sccm | 0 | 2.5 | 0 |
| Plasmaleistung / W | 800 | 2500 | 1000 |
| Behandlungszeit / min | 10 | 4 | 10 |
| Prozessdruck / mbar | 0.022 | 0.045 | 0.032 |

### Ausführungsbeispiel Plasmapolymer C6

Für dieses Beispiel wurde Polyurethan (PU) mit einer hydrophoben ca. 220 nm dicken Plasmabeschichtung versehen. Der Wasserkontaktwinkel beträgt annähernd 110°. Die Beschichtungsparameter zeigt Tabelle 9. Die Bakterienadhäsion wurde mit dem Adhäsionsassay nach ISO 17025 bestimmt.

**Tabelle 9: Beschichtungsparameter des Ausführungsbeispiels Plasmapolymer C6**

| | Feinreinigung | Beschichtung |
|---|---|---|
| O₂-Fluss / sccm | 60 | 24 |
| HMDSO-Fluss / sccm | 0 | 70 |
| Plasmaleistung / W | 800 | 700 |
| Behandlungszeit / min | 10 | 10 |
| Prozessdruck / mbar | 0,022 | 0,034 |

### Beispiel 3: VUV-Vernetzung einer siliziumorganischen Flüssigkeit

Flache Glassubstrate (Objektträger) wurden einseitig zunächst wie in Beispiel 1 beschrieben mit der Biokompatibilitätsschicht Plasmapolymer A1 beschichtet. Anschließend wurden sie mit 270 nm flüssigem Polydimethylsiloxan-Silikonöl AK50^{®} (Fa. Wacker) versehen. Hierbei wurde das Silikonöl als Reinstoff mit Hilfe eines Aerosols aufgetragen. Danach wurde zum Maskieren die Hälfte der Glassubstrate durch eine 2 mm dicke Glasplatte abgedeckt und unter Stickstoffatmosphäre bei 1 bar mittels VUV-Strahlung der Wellenlänge 172 nm vernetzt. Hierfür wurde die gesamte Probe 6 Minuten bei einem Abstand von 10 mm von einer Excimerlampe (Hersteller: Radium Lampenwerk GmbH, Xeradex-Strahler, 172nm) mit einer Strahlungsleistungsdichte von ca. 0,82 W/cm² bestrahlt. Diese Beschichtungsvariante soll nachfolgend als Biokompatibilitätsschicht Excimer D1 bezeichnet werden.

Das unvernetzte Silikonöl unter der Maskierungsscheibe konnte ohne Probleme mit Isopropanol abgespült werden, so dass auf dieser Fläche die Biokompatibilitätsschicht Plasmapolymer A1 wieder freigelegt werden konnte.

### Beispiel 4: Biologische Beurteilung der Zelladhäsion

Im Folgenden wird ein Testverfahren beschrieben, mit dem die Biokompatibilitätsschichten biologisch beurteilt wurden. Es handelt sich hierbei um ein Färbeverfahren für an Oberflächen adhärierte Zellen, welches zu den Standardmethoden in der Zellbiologie zählt. Die auf der Oberfläche angewachsenen oder adhärenten Zellen werden hierbei mit einem Farbstoff direkt angefärbt und mikroskopisch beurteilt. Die Zahl der adhärierten Zellen kann hierbei als Zellen pro Fläche oder als Bedeckungsgrad in Prozent angegeben werden. Durch die mikroskopische Beurteilung der adhärierten Zellen ist zusätzlich möglich, die Zellen morphologisch zu beurteilen. Hierdurch kann, neben der Anzahl der Zellen pro Fläche, eine zusätzliche Beurteilung getroffen werden, wie die Biokompatibilitätsschicht auf die Zellen wirkt. Für die Betrachtung von Materialien, die nicht transparent sind, muss dies mit einem Auflichtmikroskop erfolgen (z.B. das Imager M1 Mikroskop von Zeiss).

Für die unten aufgeführten Testungen wurde die in der EN ISO 10993-5: 1999 für die Prüfung von Medizinprodukten u.a. empfohlene Zelllinie V-79 verwendet. Zum Anfärben der adhärierten Zellen wurde der in der medizinischen Histologie häufig verwendete Farbstoff Eosin-Hämatoxylin eingesetzt. Es können aber auch Vitalfarbstoffe wie z.B. das Neutralrot verwendet werden, die zusätzlich noch eine Aussage zur Vitalität der Zellen geben können. Durch die Menge des aufgenommenen bzw. gebundenen Farbstoffs kann zwischen lebenden, geschädigten und toten Zellen unterschieden werden. Die Verwendung von Eosin-Hämatoxylin zum Anfärben der Zellen hat den zusätzlichen Vorteil, dass dieser Farbstoff die Zellen nicht nur im sichtbaren Licht, sondern auch im Fluoreszenz-Bereich anfärbt. Mit Hilfe eines Fluoreszenzmikroskops können hierdurch auch solche Zellen sichtbar gemacht werden, die aufgrund der Substratbeschaffenheit nicht oder nur schlecht im Lichtmikroskop beurteilt werden können.

Die Versuche wurden wie nachfolgend beschrieben durchgeführt. Die Vorbereitung der Zellstammkultur erfolgte hierbei in Anlehnung an die EN ISO 10993-5: 99. Die V79-Zellen werden in 96 × 21 mm Zellkulturschalen, mit einer Wachstumsfläche von 60,1 cm³ (TPP® Techno Plastic Products AG) bei 37 °C, 5%-CO₂-Gehalt und wasserdampfgesättigter Atmosphäre im Brutschrank mit HAM's F12-Medium (10% Fötales Kälber Serum [FKS]) kultiviert. Bei etwa 80% Konfluenz (Bedeckung nach 3-4 Tage) erfolgte eine Passage der Zellen. Zur Herstellung einer Zellsuspension wurde zunächst das alte Medium aus den Schalen abgesaugt. Anschließend wurden die Schalen mit je 2 ml phosphat-gepufferter Salzlösung (PBS) gewaschen. Dann erfolgte ein erneutes Waschen mit 0,5 ml einer unverdünnten Trypsin-Lösung (vom Schweine-Pankreas, SIGMA-Aldrich) und Absaugen dieser Lösung. Anschließend wurden 3-4 Tropfen der unverdünnten Trypsin-Lösung auf die Zellen gegeben und für ca. 5 Minuten im Brutschrank inkubiert. Durch diese Behandlung lösen sich die Zellen von der Oberfläche ab und waren im Mikroskop als runde Zellen sichtbar. Um die enzymatische Reaktion abzustoppen, wurden 2 ml Serumhaltiges Kulturmedium hinzugegeben (HAM's F12).

Die Zellsuspension wurde durch vorsichtiges Auf- und Abpipettieren mit einer Pasteurpipette gut gemischt. Hierdurch wurde gewährleistet, dass sich auch die letzten Zellcluster auflösten. Anschließend erfolgte mit Hilfe einer Neubauer Zählkammer die Bestimmung der Zellen pro ml und die Einstellung der benötigten Zellzahl für die Versuche.

Die zu testenden, mit der Biokompatibilitätsbeschichtung versehenen Materialien wurden in die Vertiefungen einer 6-Lochzellkulturplatte (9,6 cm² Wachstumsfläche pro Vertiefung) überführt, wobei die Materialien vorher mit 70%igem Isopropanol, PBS und Nährmedium gewaschen wurden. Pro Vertiefung wurden dann 4 ml Zellsuspension mit 87.500 Zellen pro ml in Ham's F12 Medium mit 10% Serum hinzupipettiert. Pro Vertiefung waren somit insgesamt 350.000 V79 Zellen vorhanden. Anschließend wurden die Proben bei 37 °C, 5 %-CO₂-Gehalt und wasserdampfgesättigter Atmosphäre im Brutschrank für 24 h inkubiert.

Nach der Inkubation wurde das Nährmedium abgesaugt und die Proben mit je 2 ml PBS bei 50 Umdrehungen pro Minute 5 Minuten lang bei Raumtemperatur auf einem Orbitalschüttler gewaschen. Nach dem letzten Waschschritt wurde die Oberfläche der Proben mit je 1 ml einer Standard Eosin-Hämatoxylin Lösung bedeckt. Nach einer Inkubation von 3 Minuten bei 50 Umdrehungen pro Minute bei Raumtemperatur auf einem Orbitalschüttler wird die Färbelösung abgesaugt und die Proben zunächst 5 Minuten lang und danach 60 Minuten lang mit 2 ml PBS bei 50 Umdrehungen pro Minute bei Raumtemperatur gewaschen. Die Proben wurden anschließend mit Hilfe eines Auflichtmikroskops (Zeiss Axio Imager.M1) betrachtet und digitale Bilder (AxioCam MRC von Zeiss) aufgenommen. Für Substrate, die eine sehr dunkle Oberfläche besaßen und dadurch nur schlecht im Lichtmikroskop zu betrachten waren, wurde mit Hilfe von Fluoreszenzbeleuchtung und entsprechender Filtersätze - integrierte Bestandteile des Axio Imager.M1 Mikroskopes - Fluoreszenzbilder aufgenommen. Zur biologischen Beurteilung der Biokompatibilitätsschicht wurde zum einen die Morphologie der adhärierten Zellen bewertet. Bei einer die Zelladhäsion fördernden Oberfläche lagen die Zellen adhärent und gestreckt vor. Für Beschichtungen mit reduzierter Adhäsion lagen entweder keine Zellen vor oder solche, die eine abweichende Morphologie aufweisen. Zum anderen wurde mit Hilfe einer Bildverarbeitungssoftware (ImageJ) der Bedeckungsgrad berechnet. Bei einer Erhöhung des Bedeckungsgrades um 40 bis 80% im Vergleich zum unbehandelten Substrat liegt eine verbesserte Zelladhäsion vor, bei einer Erhöhung um mehr als 80% eine stark verbesserte Zelladhäsion. Bei einer Verringerung des Bedeckungsgrades um 40 bis 80% im Vergleich zum unbehandelten Substrat liegt eine reduzierte Adhäsion vor, bei einer Verringerung um mehr als 80% eine stark reduzierte Zelladhäsion.

**Tabelle 10: Bewertungsmatrix der Zelladhäsion**

| **Zelladhäsion** | **Änderung des Bedeckungsgrads** | **Morphologie** |
|---|---|---|
| verbessert | 40 % bis 80 % erhöht | adhärent, gestreckt |
| Stark verbessert | mehr als 80 % erhöht | adhärent, gestreckt |
| reduziert | 40 % bis 80 % verringert | kugelig-rund |
| Stark reduziert | mehr als 80 % verringert | kugelig-rund |

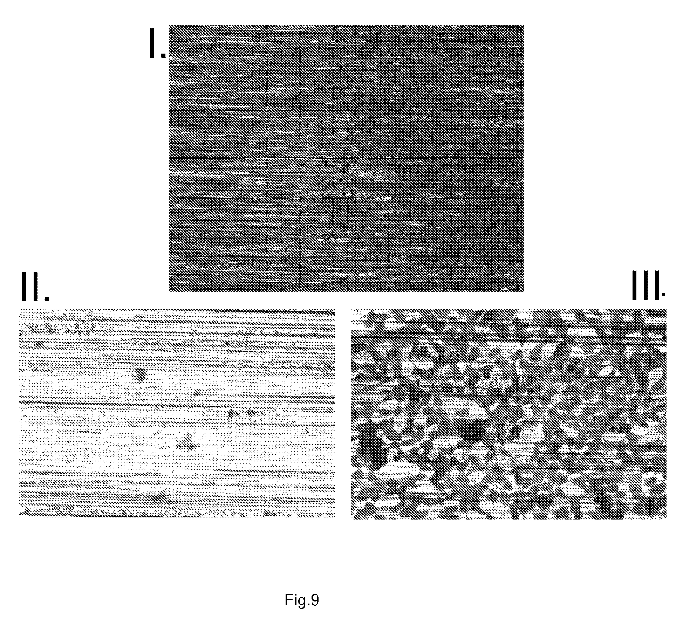

Die Ergebnisse der nachfolgend beschriebenen Versuche mit unterschiedlichen Materialien und Biokompatibilitätsbeschichtungen zeigen deutlich, dass mit Hilfe der Beschichtung unterschiedliche Oberflächeneigenschaften auf den Materialien erzielt werden können. Beispielhaft wird anhand der Figuren 9 bis 16 näher auf einige repräsentative Ergebnisse eingegangen.

Die Figur 9 zeigt unter I. ein Aluminiumplättchen, welches links mit der anti-adhäsiven Biokompatibilitätsschicht Plasmapolymer A1 und rechts mit der adhäsions-fördernden Biokompatibilitätsschicht Plasmapolymer B2 beschichtet wurde. Hierfür wurde zunächst Plasmapolymer A1 aufgebracht, dann die linke Hälfte der Probe mit einem Edelstahlplättchen abgedeckt (maskiert) und anschließend Plasmapolymer B2 aufgebracht. Unter II. wird ein Ausschnittsbild der linken Probenseite und unter III. ein Ausschnittsbild der rechten Probenseite gezeigt. Die adhärenten Fibroblastenzellen wachsen nur auf der rechten Seite. Die Morphologie der Zellen zeigt, dass diese adhärent und gestreckt vorliegen. Auf der mit einer anti-adhäsiven Beschichtung versehenen linken Oberfläche wachsen keine Zellen.

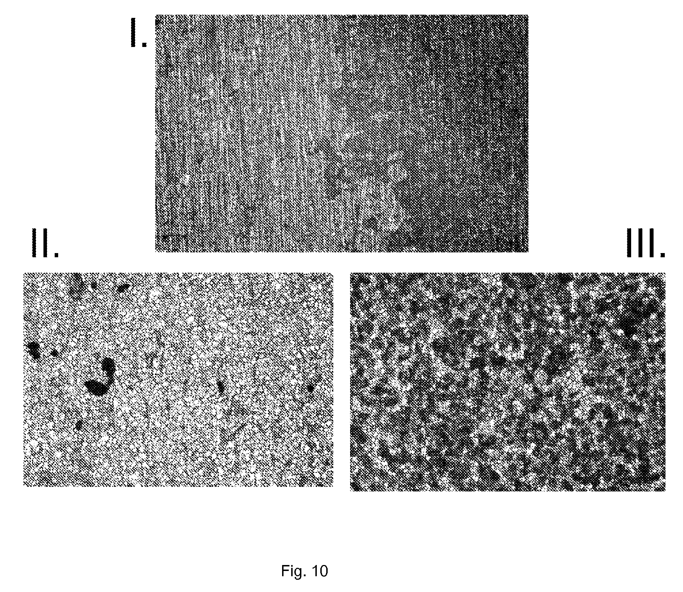

Die Figur 10 zeigt ein Substrat aus Edelstahl, welches links mit der anti-adhäsiven Biokompatibilitätsschicht Plasmapolymer A1 und rechts mit der adhäsions-fördernden Biokompatibilitätsschicht Plasmapolymer B2 beschichtet wurde. Hierfür wurde zunächst Plasmapolymer A1 aufgebracht, dann die linke Hälfte der Probe mit einem Edelstahlplättchen abgedeckt (maskiert) und anschließend Plasmapolymer B2 aufgebracht. Unter II. wird ein Ausschnittsbild der linken Probenseite und unter III. ein Ausschnittsbild der rechten Probenseite gezeigt. Die adhärenten Fibroblastenzellen wachsen nur auf der rechten Seite. Die Morphologie der Zellen zeigt, dass diese adhärent und gestreckt vorliegen. Die Oberfläche mit der Beschichtung für die reduzierte Zellanhaftung zeigt, dass hier nur sehr wenige Zellen zu sehen sind.

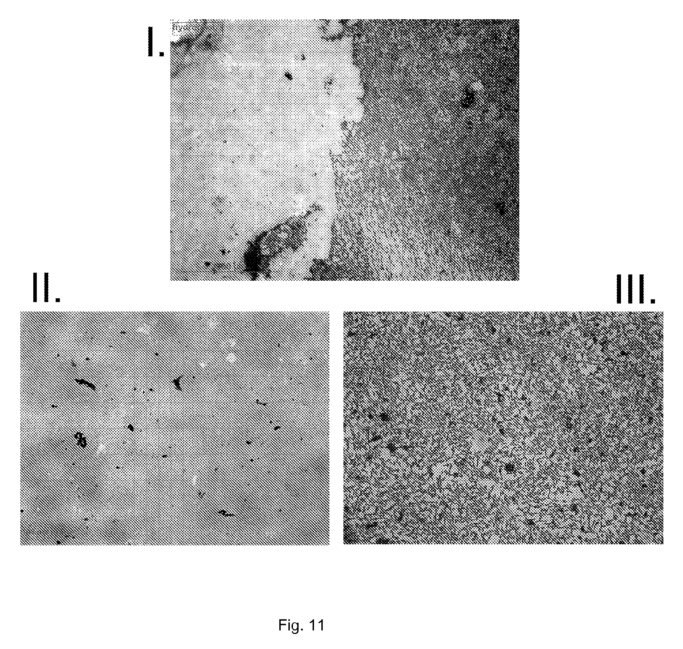

Die Figur 11 zeigt ein Glassubstrat, welches links mit der anti-adhäsiven Biokompatibilitätsschicht Plasmapolymer A1 und rechts mit der adhäsions-fördernden Biokompatibilitätsschicht Plasmapolymer B2 beschichtet wurde. Hierfür wurde zunächst Plasmapolymer A1 aufgebracht, dann die linke Hälfte der Probe mit einem Edelstahlplättchen abgedeckt (maskiert) und anschließend Plasmapolymer B2 aufgebracht. Hierdurch konnte ein unmittelbarer Übergang von Plasmapolymer A1 zu Plasmapolymer B2 realisiert werden, welcher in Figur 11 auf dem oberen Foto nach dem Anfärben der adhärierten Zellen zu sehen ist. Die adhärenten Fibroblastenzellen wachsen nur auf der rechten Seite mit dem Plasmapolymer B2, wie in der Ausschnittsvergrößerung III deutlich zu sehen ist. Die Morphologie der Zellen zeigt, dass diese adhärent und gestreckt vorliegen. Auf der linken Seite, dem Plasmapolymer A1, wie auch in der Ausschnittsvergrößerung Bild II zu sehen wachsen keine Zellen auf der Oberfläche.

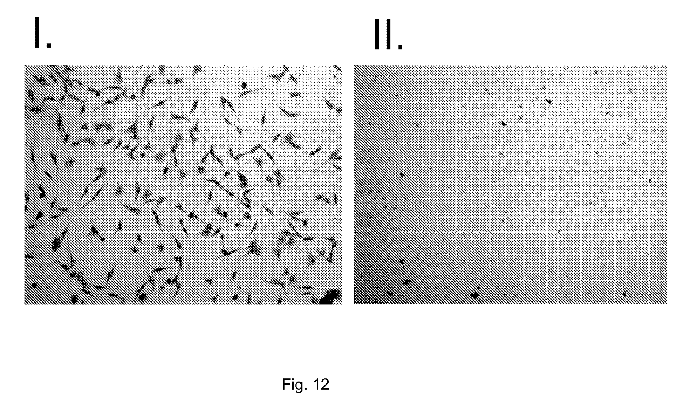

In Figur 12 sind zwei Bereiche einer Glasprobe zu sehen, welche zunächst mit der anti-adhäsiven Biokompatibilitätsschicht Plasmapolymer A1 beschichtet wurden. Anschließend wurde die Probe mit Silikonöl belegt, eine Hälfte mit einem Glas-Objektträger abgedeckt und die Probe wie oben beschrieben zur Erzeugung der Biokompatibilitätsschicht Excimer D1 mit Hilfe einer Excimerlampe vernetzt. Das Silikonöl unter dem Objektträger blieb unvernetzt und wurde mit Isopropanol abgewischt. Hierdurch resultierte eine Probe mit einer lokal abgegrenzten Fläche mit der anti-adhäsiven Biokompatibilitätsschicht Plasmapolymer A1, zu sehen in Bild II, und einer lokal abgegrenzten Fläche mit der Biokompatibilitätsschicht Excimer D1, zu sehen in Bild I. Auf der Fläche mit der freiliegenden anti-adhäsiven Biokompatibilitätsschicht Plasmapolymer A1 wachsen die Zellen gar nicht an. Auf der Fläche mit der Biokompatibilitätsschicht Excimer D1 wachsen die Zellen hingegen gut an.

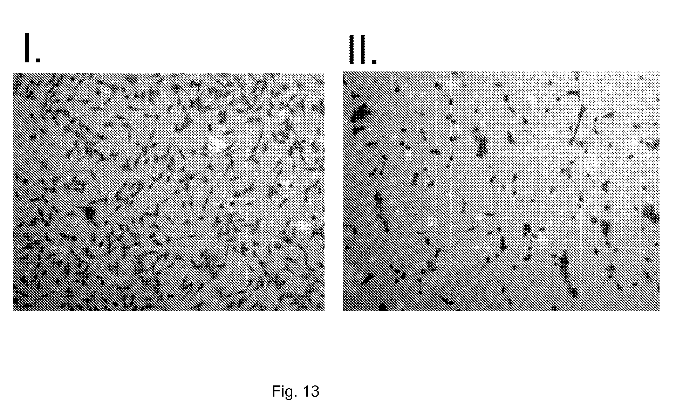

In Figur 13 sind Keramikproben zu sehen, in Bild I. unbehandelt und in Bild II. mit der anti-adhäsiven Biokompatibilitätsschicht Plasmapolymer A1 versehen. Auf der Probe in Bild II, die mit der anti-adhäsiven Beschichtung versehen ist, wachsen die Zellen reduziert an. Auf der unbehandelten Probe wachsen die Zellen hingegen gut an.

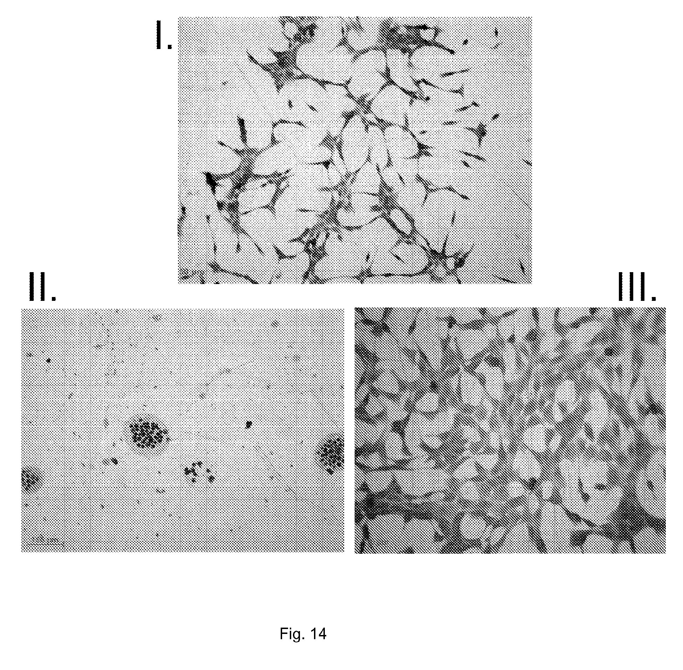

In Figur 14 sind PMMA-Kunststoffproben zu sehen, die mit unterschiedlichen Beschichtungen versehen wurden. Im oberen Bild I ist die unbehandelte Probe zu sehen, bei der die Zelladhäsion nur mittelmäßig ausgeprägt ist. In Bild III ist die mit der adhäsionsfördernden Biokompatibilitätsschicht Plasmapolymer B2 versehene Probe zu sehen; hier wachsen die Zellen verbessert an. Die Morphologie der Zellen zeigt, dass diese adhärent und gestreckt vorliegen. In Bild II. ist die mit der anti-adhäsiven Biokompatibilitätsschicht Plasmapolymer A1 versehene Probe zu sehen. Hier wachsen nur wenige bis keine Zellen.

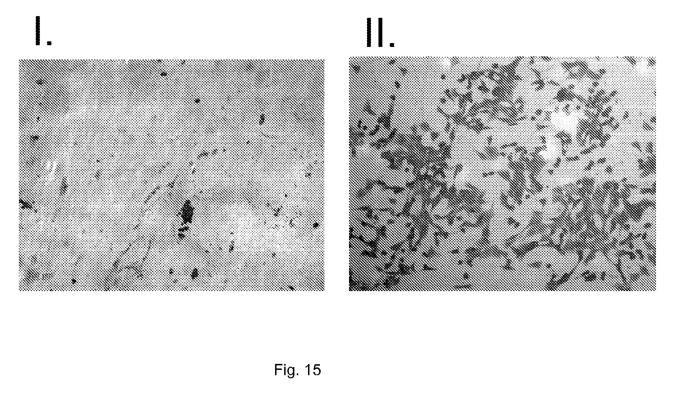

In Figur 15 sind Silikonproben zu sehen, in Bild I. die unbehandelte Referenz und in Bild II. mit der adhäsions-fördernden Biokompatibilitätsschicht Plasmapolymer C1 versehen. Auf der Probe Bild II., die mit der adhäsions-fördernden Beschichtung versehen ist, wachsen die Zellen verbessert an. Die Morphologie der Zellen zeigt, dass diese adhärent und gestreckt vorliegen. Auf der unbehandelten Probe Bild I. wachsen nur sehr wenige bis keine Zellen auf der Oberfläche.

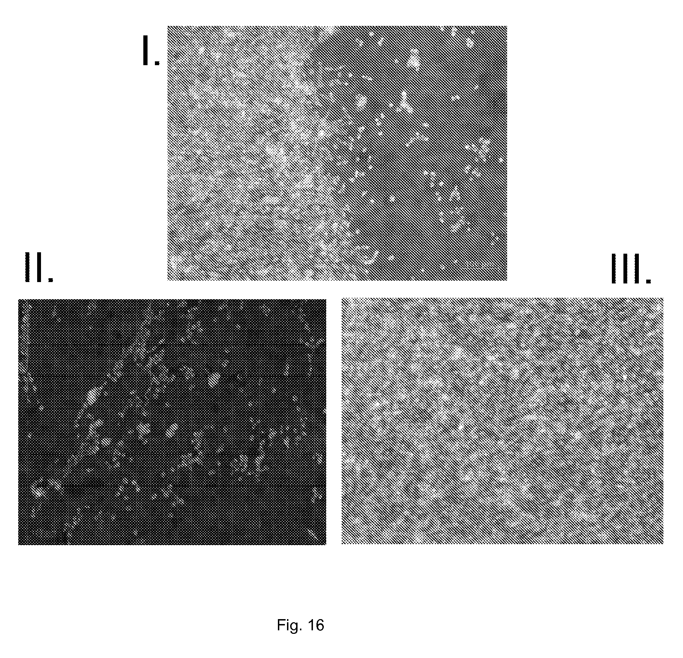

Die Figur 16 zeigt ein Substrat aus Titan, welches links unbehandelt ist (Bild I u. III) und rechts mit der anti-adhäsiven Biokompatibilitätsschicht Plasmapolymer A1 beschichtet wurde (Bild I u. II). Die Zellen wurden zur Sichtbarmachung im Fluoreszenzmikroskop betracht und das rote Farbbild in ein Graustufenbild umgewandelt.

Die Ergebnisse zur Zelladhäsion mit allen Beschichtungsvarianten sind in Tabelle 11 angegeben.

**Tabelle 11: Ergebnisse der Zelladhäsion auf unterschiedlichen Materialien und Beschichtungen im Vergleich zu den unbehandelten Materialien**

| **Substrat** | **Beschichtung (Aus Beispiel 1, 2 oder 3)** | **Änderung der Zelladhäsion** |
|---|---|---|
| Aluminium | Plasmapolymer A1 | stark reduziert |
| Aluminium | Plasmapolymer B2 | nahezu gleich bleibend (gute Zelladhäsion) |
| Edelstahl | Plasmapolymer A1 | stark reduziert |
| Edelstahl | Plasmapolymer B2 | nahezu gleich bleibend (gute Zelladhäsion) |
| Glas | Plasmapolymer A1 | stark reduziert |
| Glas | Plasmapolymer A2 | stark reduziert |
| Glas | Plasmapolymer A3 | stark reduziert |
| Glas | Plasmapolymer B2 | nahezu gleich bleibend (gute Zelladhäsion) |
| Glas | Plasmapolymer B3 | nahezu gleich bleibend (gute Zelladhäsion) |
| Glas | Excimer D1 | nahezu gleich bleibend (gute Zelladhäsion) |
| Keramik | Plasmapolymer A1 | stark reduziert |
| Keramik | Plasmapolymer B1 | nahezu gleich bleibend (gute Zelladhäsion) |
| Polymethylmethacrylat (PMMA) | Plasmapolymer A1 Plasmapolymer A1 | stark reduziert stark reduziert |
| Polymethylmethacrylat (PMMA) | Plasmapolymer B2 Plasmapolymer B2 | Verbessert |
| Silikon | Plasmapolymer A1 | nahezu gleich bleibend (schlechte Zelladhäsion) |
| Silikon | Plasmapolymer C1 | stark verbessert |
| Titan | Plasmapolymer A1 | stark reduziert |

### Biologische Beurteilung der Bakterienadhäsion

Tabelle 12 zeigt die relative Bakterienadhäsion der Biokompatibilitätsschicht C5, bezogen auf unbeschichtetes Polyurethan (PU) mit definitionsgemäß 100% Adhäsion. Durch eine hydrophile Ausstattung der Oberfläche (Plasmapolymer C5) kann die Ädhäsion von gram-negativen E.Coli auf 66% gesenkt werden. Gleichzeitig nimmt sogar die Adhäsion von gram-positiven Staphylococcus epidermidis ab. Mit Hilfe der hydrophilen Beschichtung ist es möglich, dass bei beiden Bakterienarten die Adhäsion verringert wird.

**Tabelle 12: Relative Veränderung der Bakterienadhäsion**

| | Escherichia coli | Staphylococcus epidermidis |
|---|---|---|
| PU | 100% | 100% |
| PU+Beschichtung C5 | 66% | 83% |

Tabelle 13 zeigt die relative Bakterienadhäsion der Biokompatibilitätsschicht C6, bezogen auf unbeschichtetes PU mit definitionsgemäß 100% Adhäsion. Durch eine hydrophobe Ausstattung der Oberfläche (Plasmapolymer C 6) kann die Adhäsion von gram-positiven Staphylococcus epidermidis auf 56 % gesenkt werden. Die Adhäsion von E. Coli hingegen wird nicht wesentlich verringert.

**Tabelle 13: Relative Veränderung der Bakterienadhäsion**

| | Escherichia coli | Staphylococcus epidermidis |
|---|---|---|
| PU | 100% | 100% |
| PU+Beschichtung C6 | 92% | 56% |

### Anwendungsbeispiele

### Anwendungsbeispiel 1: Oxigenatormembran

Polypropylen-Oxygenatorfasern wurden mit den hydrophilen Plasmapolymerschichten C1, C2, C3 und C4 beschichtet. Sauerstoffarmes Blut, welches in Kontakt mit den so beschichteten Fasern war, konnte Sauerstoff so effizient aufnehmen wie bei Verwendung der unbeschichteten Polypropylen-Oxygenator-Fasern. Anhand von Blutuntersuchungen konnte gezeigt werden, dass die Bildung von Thromben im Vergleich zu unbeschichteten Fasern deutlich reduziert worden ist.

### Anwendungsbeispiel 2: Implantate

Knochenfixationsnägel werden mit Bereichen mit reduzierter Zellanbindung (Plasmapolymerschicht A1) versehen, wie zu Figur 9 beschrieben maskiert, und anschließend mit Plasmapolymerschicht B2 versehen. Beide Bereiche (A1 und B2) werden wie in Beispiel 4 beschrieben auf ihre Biokompatibilität geprüft und als biokompatibel befunden. An der mit Plasmapolymer A1 beschichteten Fläche war die Zelladhäsion wie oben beschrieben gering, während sie an der mit B2 beschichteten Fläche wie beschrieben der von Edelstahl entsprach. Vergleichbare Befunde ergeben sich bei entsprechender Beschichtung eines Hüftgelenks, medizinischen Nagels, Klammer, Faden und/oder Schraube.

### Anwendungsbeispiel 3: Gefäße (Arzneimittelbehältnisse) für Körperflüssigkeit, Gewebe, Biomoleküle, Pharmazeutika

Transport- bzw. Aufbewahrungsgefäße aus Glas für verdünnte wässrige Proteinlösungen können mit der Biokompatibilitätschicht Plasmapolymer A1 (gemäß Beispiel 1) beschichtet werden. Hierdurch wird eine deutliche Reduzierung der Anhaftung der Proteine erreicht. Dadurch kann zum einen die benötigte Ausgangskonzentration der Proteine deutlich reduziert werden. Zum anderen werden die Schwankungen in der realen Konzentration der Proteine verringert. Bei den Proteinen handelt es sich um therapeutisch wirksame Antikörper.

### Anwendungsbeispiel 4: Wundauflagen

Wundauflagen wurden auf eine Träger-Polymerfolie geklebt, welche in einem Bahnwarenprozess innerhalb des Plasmareaktors von Rolle zu Rolle gewickelt werden. Dabei wurde die Biokompatibilitätsschicht Plasmapolymer A1 (gemäß Beispiel 1) aufgetragen. Die Schichtdicke auf Referenzsubstraten wurde mit ca. 50 nm bestimmt. Durch diese Plasmabeschichtung wird das Verkleben der Wundauflage durch während des Wundverschlusses neu gebildete Zellen vermindert, ein Gas- und Flüssigkeitsaustausch jedoch gewährleistet.

### Anwendungsbeispiel 5: Katheter (Blasenkatheter, Koronarkatheter)

Katheter werden im Bereich der außenliegenden Oberflächen in einem Plasmareaktor beschichtet. Hierzu werden die Katheter in einem Niederdruck-Plasmaprozess beschichtet. Bei Verwendung einer Biokompatibilitätsschicht Plasmapolymer A1 wird das Ein- und Ausführen der Katheter durch die veränderten Oberflächeneigenschaften verbessert.

### Langzeitwirkung

Bei Verwendung der Biokompatibilitätsschicht Plasmapolymer B2 (gemäß Beispiel 1) führt die Beschichtung dazu, dass der Katheter sich mit dem Gewebe besser verbinden lässt. Hierdurch kann z.B. erreicht werden, dass ein Langzeit-Katheter nicht so leicht verrutscht.

Zudem wurden Insulinkatheter aus Edelstahl mit der Biokompatibilitätsschicht Plasmapolymer A1 ausgestattet. Hierdurch kann das Zuwachsen des Insulinkatheters reduziert und somit die Standzeit des Katheters verlängert werden. Durch die Beschichtungen wird zudem die Hämokompatibilität erhöht.

### Anwendungsbeispiel 6: Angioplasieballon

Bei Verwendung einer Biokompatibilitätsschicht Plasmapolymer A1 (gemäß Beispiel 1) kann das Ein- und Ausführen des Angioplasieballon durch die veränderten Oberflächeneigenschaften erleichtert werden, da durch die veränderte Oberflächenspannung die Reibung vermindert werden kann. Dieses schont die bei diesem Eingriff betroffenen Gefäßareale.

### Anwendungsbeispiel 7: Blutgefäßstents

Blutgefäßstents können zunächst mit der Biokompatibilitätsschicht Plasmapolymer A1 (gemäß Beispiel 1) versehen werden. Anschließend kann die Außenseite mit Hilfe von UV-Strahlung oberflächlich oxidiert werden. Hierzu werden die Stents auf einem rotierenden Dorn bei einem Abstand von 10 mm unter einer Stickstoffatmosphäre bei einem Druck von 1 bar mit einem Feuchtigkeitsgehalt von ca. 1% mit dem Licht der Wellenlänge 172 nm einer Excimerlampe (Xeradex-Strahler, 172 nm, Radium Lampenwerk GmbH) ausgesetzt. Die Bestrahlung erfolgt beispielsweise über 30 Sekunden mit einer Strahlungsleistungsdichte von ca. 0,82 W/cm².

Durch diese Behandlung wird die Zelladhäsion auf der Innenseite des Stents deutlich verringert, während die Außenseite weiterhin eine gute Zelladhäsion ermöglicht. Durch die verbesserte Zelladhäsion auf der Außenseite kann der Spalt, der sich durch die Stentdilatation bildet, schneller geschlossen werden, da Zellen/ Gewebe auf dem Material anwachsen können. Eine innenliegende Behandlung der Oberfläche mit einer Biokompatibilitätsschicht des Typs A1 verhindert hingegen, dass sich Blutzellen bzw. Blutbestandteile an diesen Bereich anlagern und zu einer Verstopfung des Stents führen.

### Anwendungsbeispiel 8: Kanüle

Kanülen können mit der Biokompatibilitätsschicht Plasmapolymer A1 (gemäß Beispiel 1) versehen werden. Durch die Behandlung mit der Biokompatibilitätsschicht lassen sich die Kanülen leichter in Gewebe oder Gefäße platzieren bzw. ebenso leichter wieder entfernen.

### Anwendungsbeispiel 9: Injektionssystem

Kanülen können mit der Biokompatibilitätsschicht Plasmapolymer B2 (gemäß Beispiel 1) versehen werden. Durch die Behandlung mit der Biokompatibilitätsschicht wird erreicht, dass sich im Bereich des Gewebedurchtritts die Zellen besser an das Injektionsmaterial anlagern. Hierdurch wird eine verbesserte Wundheilung/ Verschluss erreicht. Das Eindringen von Krankheitserregern kann hierdurch stark reduziert werden.

### Anwendungsbeispiel 10: künstliches Organ

Als Transplantate werden z.B. künstliche Nieren oder Herzen seit einiger Zeit eingesetzt. Ein schwerwiegendes Problem ist hierbei, dass der Körper stark auf z.B. den Kunststoff dieser Transplantate reagiert. Durch eine Biokompatibilitätsschicht wie beispielsweise Plasmapolymer B2 können hierbei diese starken körpereigenen Reaktionen vermindert werden. Auf der anderen Seite ist es durch eine Biokompatibilitätsschicht wie beispielsweise Plasmapolymer A1 möglich zu verhindern, dass bestimmte Bereiche der künstlichen Organe durch ungewolltes Zellwachstum in ihrer Funktion beeinträchtigt werden.

### Anwendungsbeispiel 11: Herzschrittmacher und eine Stromquelle desselben

Herzschrittmacher können zusammen mit Elektroden, Kabeln und Gehäusen zunächst mit einer Biokompatibilitätsschicht wie beispielsweise Plasmapolymer A1 (gemäß Beispiel 1) ausgestattet werden. Anschließend werden die Elektroden und Kabel mit Hilfe eines Sauerstoff-haltigen Plasmas aus einer Atmosphärendruck-Plasmadüse wie beispielsweise der Plasmadüse PFW10 der Firma PlasmaTreat oberflächlich oxidiert. Zur Erzeugung eines Plasma-Strahls wird beispielsweise Luft mit einem Volumenstrom von ca. 1400 L/h durch die Plasmadüse geleitet und innerhalb der Düse ein gepulstes Plasma (Pulsdauer ca. 50 µs, Puls/Pause-Verhältnis ca. 1/3) mit einer Frequenz von ca. 18 kHz, einer Elektrodenspannung von ca. 10 kV und einer Plasmaleistung von ca. 0,8 kW erzeugt. Für die Behandlung wird beispielsweise ein Abstand von ca. 10 mm zwischen der Plasmadüse und der Oberfläche eingestellt. Die Behandlung erfolgt beispielsweise mit einer Relativgeschwindigkeit (zwischen Oberfläche und Düse) von 16 m/min.

Hierdurch kann die Verankerung der Elektroden und Kabel im Gewebe verbessert werden. Zudem wird es zu einer Verminderung des Einwachsens des Gehäuses durch umliegendes Gewebe kommen und die Biokompatibilität z.B. der Elektroden erhöht.

### Anwendungsbeispiel 12: Prothese (offene Implantate, Epithesen)

Prothesen, die vollständig von Gewebe umschlossen sind (so genannte Endoprothesen), werden im Anwendungsbeispiel 2 "Implantate" aufgeführt. Bei so genannten offenen Implantaten befindet sich ein Teil im Körpergewebe, während sich der andere Teil außerhalb befindet. Im Bereich des Durchtritts durch die Haut bzw. das Gewebe erhöht eine Biokompatibilitätsschicht des Typs Plasmapolymer B2 den Wundverschluss, da hier die Gewebezellen verbessert anwachsen. Durch diese Beschichtung wird zusätzlich die Gewebeverträglichkeit der offenen Prothese verbessert. Zusätzlich wird hierdurch verhindert, dass Bakterien über die Durchtrittsbereiche in den Körper gelangen und Infektionen auslösen, da diese Bereiche einen verbesserten Verschluss zwischen Gewebe und Prothesenmaterial haben.

### Anwendungsbeispiel 13: Cochlea -Implantat

Im Bereich der Cochlea-Implantate können Biokompatilitätsbeschichtungen des Typs Plasmapolymer B2 der dünnen Anschlusskabel dabei helfen, dass diese besser am Felsenbein fixiert werden. Dieses hilft dabei, dass das Elektrodenset nicht so leicht herausrutscht. Zusätzlich sorgt diese Beschichtung dafür, dass sich das Implantat besser in das umgebende Gewebe integriert. Hierdurch können u.a. Unverträglichkeiten gegenüber den Implantatmaterialien (z.B. dem Silikon) verhindert werden, welches einen schnelleren Heilungsprozess ermöglicht und Entzündungsprozesse verhindert.

### Anwendungsbeispiel 14: künstliche Herzklappe

Durch die Beschichtung der künstlichen Klappenoberfläche mit einer Biokompatibilitätsschicht Plasmapolymer A1 (gemäß Beispiel 1) kann die Neigung zur Bildung von Thromben reduziert werden.

### Anwendungsbeispiel 15: Herzklappenring

Durch eine Beschichtung des Herzklappenrings mit einer Biokompatibilitätsschicht Plasmapolymer B2 kann dieser verbessert in das umliegende Herzgewebe integriert werden.

### Anwendungsbeispiel 16: Intraocularlinse

Durch die Beschichtung einer Intraocularlinse mit einer plasmapolymeren Beschichtung des Typs B2 kann die Biokompatibilität erhöht werden, hierdurch wird das Einwachsen und die Benetzbarkeit der Intraocularlinse verbessert.

### Anwendungsbeispiel 17: Blutkonserven-Beutel

Beim Transport oder der Lagerung von Blut und Blutbestandteilen muss verhindert werden, dass diese mit der Oberfläche des Beutelmaterials reagieren, da hierdurch unerwünschte Reaktionen ausgelöst werden könnten. Durch die Behandlung des innenliegenden Beutelmaterials mit einer Biokompatibilitätsschicht Plasmapolymer A1 kann eine solche unerwünschte Reaktion vermindert werden.

### Anwendungsbeispiel 18: Zellkultur-Behältnis

Durch die Behandlung von Zellkulturschalen (Petrischalen, 6-, 12-, 24- und 96-Loch Platten) mit einer Biokompatibilitätsschicht des Typs B2 kann erreicht werden, dass die Zellen besser der Zellkultur-Behältnisoberfläche anwachsen. Dies hat entscheidende Vorteile für zellbiologische und Zytotoxizitäts Versuche.

Durch eine Biokompatibilitätsschicht Plasmapolymer A1 kann, bei z.B. Versuchen mit Blutzellen, verhindert werden, dass eine unerwünschte Reaktion dieser Zellen mit dem Testgefäß stattfindet, wie z.B. eine ungewollte Immunreaktion.

### Anwendungsbeispiel 19: Fermenter

Im Bereich der Fermenter kann eine Biokompatibilitätsschicht des Typs B2 dazu dienen, den Bewuchs von Oberflächenstrukturen im Fermenter zu verbessern. So lassen sich hierdurch zum Beispiel Zellen, die z.B. bestimmte Proteine bilden und in das diese Zellen umgebende Medium abscheiden, auf den Fermenteroberflächenmaterialien (Waben, Lamellen, 3D-Strukkturen u.a.) verbessert anlagern. Bei diesen Zellen handelt sich um Protein-produzierende CHO-Zellen. Hierdurch kann die Menge an gebildeter Protein-Substanz erhöht werden.

### Anwendungsbeispiel 20: Sensor

Auf Sensoren, die mit einer Biokompatibilitätsschicht Plasmapolymer A1 versehen sind, werden sich im Bereich der Sensorfläche weniger bzw. keine unerwünschten Zellen oder Biomoleküle anlagern. Durch eine Ablagerung im Bereich des Sensors kommt es häufig zum Versagen dieser Messinstrumente. Durch eine Biokompatibilitätsschicht Plasmapolymer A1 wird eine verlängerte Funktionsfähigkeit dieser Sensoren erreicht.

### Anwendungsbeispiel 21: Sonden

Perkutane Endoskopische Gastrostomie Sonden (oder weitere wie z.B die Perkutane Endoskopische Jejunostomie-Sonde) dienen der Versorgung von Patienten mit Nahrung und Flüssigkeiten. Im Bereich des Durchtritts durch den Magen bzw. Darm kann es zu Undichtigkeiten kommen, die dazu führen, dass sich der Magen/Darm-Inhalt in den Bauchraum ergießt und es zu lebensbedrohlichen Bauchfellentzündungen kommt. Durch eine Biokompatibilitätsschicht des Typs B2 kann sich das Magen/Darmgewebe verbessert an das Sondenmaterial anlagern und hierdurch Undichtigkeiten verringert werden.

### Anwendungsbeispiel 22: Filtermaterial

Durch die Beschichtung von Filtermaterial mit Hilfe einer Biokompatibilitätsschicht Plasmapolymer B2 kann erreicht werden, dass sich hier Bakterienzellen verbessert anheften können. Die hier angehefteten Zellen können dazu verwendet werden, z.B. bestimmte Substanzen wie organische Kontaminationen aus einer diese Zellen umströmenden Flüssigkeit oder Gasen herauszufiltern bzw. abzubauen. Anwendungen als Biofilter können hierdurch ermöglicht werden.

### Anwendungsbeispiel 23: Biokompatible Trägermatrices (engl. scaffolds,)

Im Bereich der in vitro Gewebezüchtung werden zunehmend Trägermaterialien auf Basis von Keramiken oder Polymermaterialien verwendet. Für diese Trägermaterialien ist von zentraler Bedeutung, dass autologe Zellen auf diesen Trägermaterialien anwachsen. Dies kann beispielsweise mit der Biokompatibilitätsschicht Plasmapolymer B2 erleichtert werden.

## Patentansprüche

1. Verwendung einer vernetzten siliziumhaltigen Schicht enthaltend, bestehend im wesentlichen aus oder bestehend aus Silizium, O, C, H, optional N, die durch Plasmapolymerisation und/oder Vernetzung von siliziumorganischen Flüssigkeiten mit Hilfe eines Plasmaprozesses und/oder UV-Strahlung einer Wellenlänge unter 250 nm ohne die Verwendung von Metallen einer Ordnungszahl über 14 herstellbar ist, mit einem atomaren Verhältnis von Sauerstoff zu Silizium von 0,75 bis 2,2 und einem atomaren Verhältnis von Kohlenstoff zu Silizium von 0,1 bis 2,5, gemessen mit XPS,
- als biokompatible Oberfläche oder
- zum Vermitteln oder Versehen einer Oberfläche mit einer biokompatiblen Wirkung, oder
- zum Modifizieren einer biokompatiblen Wirkung einer Oberfläche, oder
- zur definierten Beeinflussung der Adhäsion von Biomolekülen und/oder Zellen (Pro- oder Eukaryonten).

2. Biokompatibler Gegenstand, umfassend einen Oberflächenbereich mit einer vernetzten siliziumhaltigen Schicht enthaltend, bestehend im wesentlichen aus oder bestehend aus Silizium, O, C, H, optional N, die durch Plasmapolymerisation und/oder Vernetzung von siliziumorganischen Flüssigkeiten mit Hilfe eines Plasmaprozesses und/oder UV-Strahlung einer Wellenlänge unter 250 nm ohne die Verwendung von Metallen einer Ordnungszahl über 14 herstellbar ist, mit einem atomaren Verhältnis von Sauerstoff zu Silizium von 0,75 bis 2,2 und einem atomaren Verhältnis von Kohlenstoff zu Silizium von 0,1 bis 2,5, gemessen mit XPS (Biokompatibilitäts-Oberfläche), wobei der Gegenstand ausgewählt ist aus der Gruppe bestehend aus:
a) einem bei bestimmungsgemäßem Gebrauch mit Körperflüssigkeit eines Lebewesens oder einem ihrer Bestandteile umströmten oder durchströmten Gegenstand,
b) einem bei bestimmungsgemäßem Gebrauch implantierten Gegenstand,
c) einem Behältnis zur Aufnahme und/oder zum Transport von Körperflüssigkeit, Gewebe oder deren Bestandteilen eines Lebewesens oder von Biomolekülen, bevorzugt Peptiden, Proteinen, Lipiden, Kohlenhydraten, Nukleinsäuren oder damit hergestellten Wirkstoffen.
d) einem Gegenstand zum zumindest teilweisen Bedecken von Haut oder Schleimhaut eines Lebewesens, und bevorzugt von Wunden,
e) einem bei bestimmungsgemäßem Gebrauch anderweitig mit Körperflüssig-keit, Gewebe oder deren Bestandteilen eines Lebewesens oder mit Biomolekülen, bevorzugt Peptiden, Proteinen, Lipiden, Kohlenhydraten, Nukleinsäuren oder damit hergestellten Wirkstoffen in Kontakt stehendem Gegenstand.

3. Biokompatibler Gegenstand nach Anspruch 2, **dadurch gekennzeichnet, dass** der Gegenstand einen Bereich der Biokompatibilitäts-Oberfläche besitzt mit
a) einem Wasser-Kontaktwinkel von nicht mehr als 35°, oder
b) einem Wasser-Kontaktwinkel von zumindest 90°.

4. Biokompatibler Gegenstand nach einem der Ansprüche 2 bis 3, **dadurch gekennzeichnet, dass** die siliziumhaltige Schicht der Biokompatibilitäts-Oberfläche außer aus Wasserstoff zumindest zu 98 Atom-% aus den Elementen Silizium, Kohlenstoff und Sauerstoff und typischerweise bis zu 2% Stickstoff gemessen mit XPS besteht.

5. Biokompatibler Gegenstand nach Anspruch 4, **dadurch gekennzeichnet, dass** die siliziumhaltige Schicht der Biokompatibilitäts-Oberfläche 3-15 Atom-% Kohlenstoff aufweist, bezogen auf alle Elemente außer Wasserstoff, bestimmt durch XPS.

6. Biokompatibler Gegenstand nach einem der Ansprüche 2 bis 5, **dadurch gekennzeichnet, dass** die Kohlenstoff-Atome der siliziumhaltigen Schicht der Biokompatibilitäts-Oberfläche einen Anteil von -5 - 35 % an Kohlenstoff-Atomen mit einer Bindung zu einem Sauerstoff-Atom ("CO-Kohienstoff") aufweisen, gemessen mit XPS.

7. Biokompatibler Gegenstand nach einem der Ansprüche 2 bis 6, **dadurch gekennzeichnet, dass** die Kohlenstoff-Atome der siliziumhaltigen Schicht der Biokompatibilitäts-Oberfläche einen Anteil von -5 - 20 % an Kohlenstoff-Atomen mit Bindungen zu zwei Sauerstoff-Atomen ("COO-Kohlenstoff") aufweisen, gemessen mit XPS.

8. Biokompatibler Gegenstand nach einem der Ansprüche 2 bis 7, **dadurch gekennzeichnet, dass** die siliziumhaltige Schicht der Biokompatibilitäts-Oberfläche hergestellt oder herstellbar ist durch Vernetzen eines Methylsiloxan-Precursors, vorzugsweise von Hexamethyldisiloxan durch Plasmapolymerisation, insbesondere Niederdruck- oder Atmosphärendruck-Plasmapolymerisation, oder durch Vernetzen eines Silikonöls ohne chemisch reaktive Gruppen unter Einwirkung eines Plasmas oder UV-Strahlung einer Wellenlänge unter 250 nm, insbesondere Excimer-Strahlung.

9. Biokompatibler Gegenstand nach Anspruch 8, **dadurch gekennzeichnet, dass** die siliziumhaltige Schicht der Biokompatibilitäts-Oberfläche nach dem Vernetzen des Precursors bzw. Silikonöls zumindest teilweise oxidiert wurde, vorzugsweise durch Plasma-Einwirkung, Beflammung, Oxy-Fluorierung, Laser-Behandlung oder Behandlung mit Excimer-Lampen.

10. Biokompatibler Gegenstand nach einem der Ansprüche 2 bis 9, **dadurch gekennzeichnet, dass** die siliziumhaltige Schicht der Biokompatibilitäts-Oberfläche eine Schichtdicke von höchstens 2 µm, bevorzugt 1µm, besonders bevorzugt 500 nm besitzt, sowie mindestens 5 nm, bevorzugt 10 nm, besonders bevorzugt 15 nm besitzt.

11. Biokompatibler Gegenstand nach einem der Ansprüche 2 bis 10, **dadurch gekennzeichnet, dass** die Biokompatibilitäts-Oberfläche eine oder mehrere Eigenschaften vermittelt oder verstärkt, die ausgewählt ist bzw. sind aus der Gruppe bestehend aus
- geringe oder fehlende Zytotoxizität, gemessen gemäß ISO 10993-5:2003,
- geringe oder fehlende Reizungen oder intrakutane Reaktivität, gemessen gemäß ISO 10993-10:2003,
- geringe oder fehlende systemische Toxizität (akute Toxizität, subakute Toxizität und subchronische Toxizität), gemessen gemäß ISO 10993-11:2003,
- geringe oder fehlende Genotoxizität, gemessen gemäß ISO 10993-3:2003,
- Implantationsverträglichkeit, gemessen gemäß ISO 10993-6:2003, und
- Hämokompatibilität, gemessen gemäß ISO 10993-4:2003.

12. Verfahren zum Herstellen eines biokompatiblen Gegenstandes nach einem der Ansprüche 2 bis 11, umfassend die Schritte
a) Bereitstellen einer vernetzten siliziumhaltigen Schicht mit einem atomaren Verhältnis von Sauerstoff zu Silizium von 0,75 bis 2,2, und einem atomaren Verhältnis von Kohlenstoff zu Silizium von 0,1 bis 2,5, gemessen mit XPS, und
b) zumindest abschnittweises Oxidieren der in Schritt a) bereitgestellten Schicht.

13. Verfahren nach Anspruch 12, **dadurch gekennzeichnet, dass** zum Begrenzen des zu oxidierenden Bereichs eine Maske, vorzugsweise eine ablösbare selbstklebende Maske, besonders bevorzugt ein Klebeband, oder eine gedruckte Maske und/oder ein vorzugsweise in Wasser zumindest teilweise lösbarer oder dispergierbarer Stoff verwendet und nach Schritt b) vorzugsweise entfernt wird.

14. Verwendung nach Anspruch 1 zum im Vergleich zu einer entsprechenden unbeschichteten Oberfläche,
- Fördern eines Anhaftens von Gewebezellen und/oder
- Reduzieren einer Anhaftung von Gewebezellen und/oder
- Reduzieren einer Anhaftung von Gewebezellen auf einem Teil der Oberfläche des biokompatiblen Gegenstands und Fördern der Anhaftung auf einem anderen Teil der Oberfläche des biokompatiblen Gegenstands und/oder
- Reduzieren einer Anhaftung pathogener Ablagerungen und/oder
- Reduzieren einer Anhaftung von Bakterien und/oder
- Reduzieren der Thrombogenität und/oder
- Reduzieren des Auftretens humoraler und zellulärer Immunreaktionen und/oder
- Reduzieren von unspezifischer Adsorption von Peptiden, Proteinen, Lipiden, Kohlenhydraten und/oder Nukleinsäuren an der Oberfläche des biokompatiblen Gegenstands und/oder
- Ermöglichen räumlich begrenzten Wachstums von Zellkulturen auf der Oberfläche des biokompatiblen Gegenstands und/oder,
- Erhöhen der Benetzbarkeit durch wässrige Flüssigkeiten, insbesondere Blut.

15. Biokompatibler Gegenstand nach einem der Ansprüche 2 bis 11, **dadurch gekennzeichnet, dass** die siliziumhaltige Schicht der Biokompatibilitäts-Oberfläche.
- bei der Analyse im XPS für die Elemente außer Wasserstoff einen
Stoffmengenanteil von
25at% bis 60at% Kohlenstoff
18at% bis 30at% Silicium
20at% bis50at% Sauerstoff
ergibt; und/oder
- ein Verhältnis von Wasserstoff zu Kohlenstoff von 1,8 : 1 bis 3,1 : 1 aufweist; und/oder
- bei der Analyse mit XPS, bei Kalibrierung auf den aliphatischen Anteil des C 1s Peaks bei 285,00 eV, im Vergleich mit einem trimethylsiloxy-terminierten Polydimethylsiloxan (PDMS) mit einer kinematischen Viskosität von 350 mm²/s bei 25 °C und einer Dichte von 0,97 g/mL bei 25 °C, der Si 2p Peak einen Bindungsenergiewert besitzt, der um maximal 0,80 eV zu höheren oder niedrigeren Bindungsenergien verschoben ist, und der 0 1s Peak einen Bindungsenergiewert besitzt, der um maximal 0,70 eV zu höheren oder niedrigeren Bindungsenergien verschoben ist.
